# EUROPEAN PATENT APPLICATION

(11) **EP 3 360 487 A2**
(43) Date of publication of application: **15.08.2018**
(21) Application number: 18150970.4
(22) Date of filing: 10.01.2018
(51) Int. Cl.: A61B 8/12, A61B 8/08, A61B 8/00

(54) **ULTRASOUND DIAGNOSTIC APPARATUS**

(30) Priority: 20.01.2017 JP 2017008149
(71) Applicant: Konica Minolta, Inc., Tokyo 100-7015 (JP)
(72) Inventor: TANIGUCHI, Tetsuya, Tokyo (JP)
(74) Representative: Atkinson, Ian Anthony

(57) **Abstract**

An ultrasound diagnostic apparatus includes the following. An ultrasound probe includes a transmitting opening including a plurality of transducers, and the transmitting opening includes a plurality of transducer groups including at least a first transducer group and a second transducer group. A transmitter generates a plurality of driving signals with different driving waveforms including at least a first driving signal and a second driving signal, provides a time delay to the plurality of driving signals so that the transmitting ultrasound focuses to a same focal point, outputs the first driving signal to the first transducer group, outputs the second driving signal to the second transducer group, and outputs the plurality of driving signals to the plurality of transducer groups of the transmitting opening. A receiver receives the receiving signal from the ultrasound probe. An image generator generates the ultrasound image data from the receiving signal.

## Description

### Background

### 1. Technological Field

The present invention relates to an ultrasound diagnostic apparatus.

### 2. Description of the Related Art

According to ultrasound diagnosis, heartbeat of the heart and movement of the fetus can be recognized by a simple operation such as placing an ultrasound probe against a body surface. Such examination is high in safety and is therefore repeatedly performed.

In such technique of displaying an ultrasound diagnostic image, it is said that images with good contrast can be obtained by imaging higher harmonic wave components in response to fundamental wave component of a transmitting signal. Such imaging method is called Tissue Harmonic Imaging (THI).

The above-described higher harmonic wave component is generated due to nonlinear distortion caused when ultrasound propagates in the subject. That is, when the ultrasound is irradiated in the live body, the signal is distorted while propagating in the tissue due to the nonlinear response of the tissue and the higher harmonic wave components increase. As a result, such receiving signal includes components such as 2f0 two times the frequency of the fundamental wave f0 and 3f0 three times the frequency of the fundamental wave.

One known method to extract the higher harmonic wave component of a receiving ultrasound in tissue harmonic imaging is a filter method. According to such method, for example, a frequency band passage filter with a central frequency of 2f0 is used to extract the higher harmonic wave component of 2f0 from the receiving signal. As another example, there is a method called pulse inversion method. According to such method, first and second transmitting waveforms with the polarity inverted from each other are transmitted with an interval in between, and phase adding is performed on the receiving signals to cancel out the fundamental wave component. With this, secondary higher harmonic wave components are emphasized. In order to cancel out the fundamental wave components, high level positive-negative driving symmetry is necessary in the transmitting driving apparatuses which invert the polarity of the waveforms.

For example, there is a known ultrasound diagnostic apparatus including the following features (for example, Japanese Patent Application Laid-Open Publication No. 2014-168555). The frequency power spectrum of the transmitting pulse signal has a frequency band included in the transmitting frequency band of -20dB in the ultrasound probe. There are strength peaks in each of the frequency side lower and higher than the central frequency in the transmitting frequency band. The strength in the frequency region between the plurality of strength peaks is -20 dB or more with the maximum value of the strength in the strength peak as the reference. The driving signal generated as described above is input in the ultrasound probe and the ultrasound image is generated by the pulse inversion method. According to such ultrasound diagnostic apparatus, penetration (invasion depth) can be enhanced while maintaining high resolution.

There is also a known ultrasound diagnostic apparatus in which the transmitting opening of the ultrasound probe is divided, and the delay profile on the inner side and the outer side is changed, and with this, the focused spot of the beam is changed in the depth direction to obtain a narrow and long beam profile (for example, Japanese Patent Application Laid-Open Publication No. 2013-158626). According to such ultrasound diagnostic apparatus, the ultrasound is transmitted to inner side = near focal spot, outer side = far focal spot.

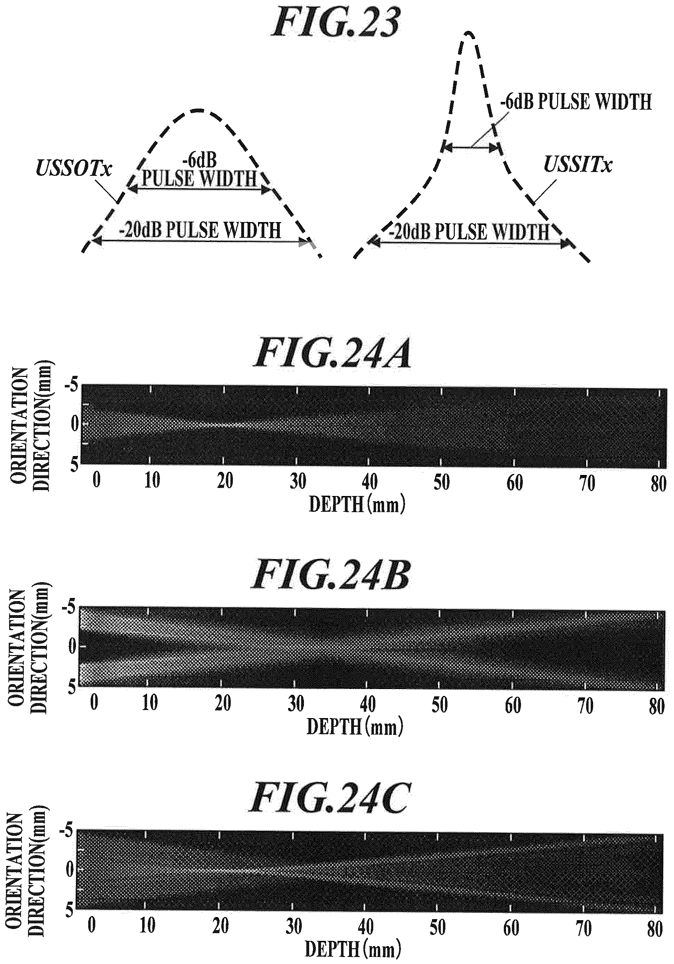

FIG. 24A is a diagram showing two dimensional sound pressure distribution of a first ultrasound beam. FIG. 24B is a diagram showing two dimensional sound pressure distribution of a second ultrasound beam. FIG. 24C is a diagram showing two dimensional sound pressure distribution of a combined ultrasound beam. Japanese Patent Application Laid-Open Publication No. 2013-158626 describes the first ultrasound beam which forms the two-dimensional sound pressure distribution (peak sound pressure [Pa]) shown in FIG. 24A is generated in the inner side portion of the transmitting opening of the ultrasound probe. The second ultrasound beam which forms the secondary sound pressure distribution shown in FIG. 24B is generated in the outer side portion of the transmitting opening of the ultrasound probe. The first ultrasound beam and the second ultrasound beam are combined to form the combined ultrasound beam, and a beam profile with a long and narrow focal point is obtained as shown in the secondary sound pressure distribution illustrated in FIG. 24C.

However, according to the ultrasound diagnostic apparatus shown in Japanese Patent Application Laid-Open Publication No. 2014-168555, a transmitting apodization mechanism is necessary to prevent acoustic noise being mixed in the shallow low receiving ultrasound portion (shallow low brightness region), and this is a reason for costs of the device becoming higher. The transmitting apodization mechanism performs the method of weighting the transmitting strength so that the amplitude of the transmitting wave of the driving signal becomes smaller towards the transducer of the ultrasound probe in the edge of the transmitting opening in the orientation direction, and with this, the side lobe is reduced. For example, the mechanism includes a substrate for transmitting apodization. Moreover, unnecessary higher harmonic waves are generated in the shallow region which does not contribute to reception, and the efficiency of generating signals which contribute to imaging compared to the sent energy is not always optimal, and the driving voltage needed to be reduced to regulate the surface temperature of the ultrasound probe on the basis of the heat emitted by resistance in the ultrasound probe due to transmitted energy.

Further, according to the ultrasound diagnostic apparatus shown in Japanese Patent Application Laid-Open Publication No. 2013-158626, although the focal point becomes narrow and long, the sound pressure near the center of the beam which is the receiving region becomes lower than the outer side in the region deeper than the transmitting focal point, and the acoustic energy is not effectively used. The S/N (SN ratio, signal to noise ratio) rather decreases. Especially, in THI which generates relying on sound pressure, a drastic reduction of sound pressure in the region deeper than the transmitting focal point means the higher harmonic waves cannot be generated in this region. Further, since the higher harmonic waves generated near the focal point is propagated mainly in the outer side region similar to the beam profile, the S/N drastically decreases than normal near the center of the beam which is the receiving region. As a result, penetration is deteriorated.

### Summary

An object of the present invention is to reduce costs and to obtain good drawing of the ultrasound image according to the received ultrasound.

To achieve at least one of the abovementioned objects, according to an aspect of the present invention, an ultrasound diagnostic apparatus reflecting one aspect of the present invention is described, the ultrasound diagnostic apparatus which uses an ultrasound probe which transmits transmitting ultrasound to a subject and which receives receiving ultrasound from the subject to generate ultrasound image data from the obtained receiving signal, the apparatus including: the ultrasound probe which includes a transmitting opening including a plurality of transducers, wherein the transmitting opening includes a plurality of transducer groups including at least a first transducer group and a second transducer group; a transmitter which generates a plurality of driving signals with different driving waveforms including at least a first driving signal and a second driving signal, provides a time delay to the plurality of driving signals so that the transmitting ultrasound focuses to a same focal point, outputs the first driving signal to the first transducer group, outputs the second driving signal to the second transducer group, and outputs the plurality of driving signals to the plurality of transducer groups of the transmitting opening; a receiver which receives the receiving signal from the ultrasound probe; and an image generator which generates the ultrasound image data from the receiving signal.

### Brief Description of the Drawings

The advantages and features provided by one or more embodiments of the invention will become more fully understood from the detailed description given hereinbelow and the appended drawings which are given by way of illustration only, and thus are not intended as a definition of the limits of the present invention.
FIG. 1 is an external diagram of an ultrasound diagnostic apparatus according to an embodiment of the present invention.
FIG. 2 is a block diagram showing a functional configuration of an ultrasound diagnostic apparatus.
FIG. 3 is a block diagram showing a functional configuration of a transmitting unit.
FIG. 4A is a diagram showing one example of a frequency characteristic of a signal strength of a transmitting ultrasound according to the present embodiment.
FIG. 4B is a diagram showing a frequency characteristic of a signal strength of a reflecting ultrasound where the depth is a surface layer.
FIG. 4C is a diagram showing a frequency characteristic of a reflecting ultrasound where the depth is near a focal point.
FIG. 4D is a diagram showing a frequency characteristic of a signal strength of a reflecting ultrasound where the depth is a portion deeper than the focal point.
FIG. 5 is a diagram showing total image quality with relation to display depth according to a present embodiment and a conventional example.
FIG. 6A is a diagram showing a time waveform of transmitting ultrasound directly after transmitting.
FIG. 6B is a diagram showing the time waveform of the transmitting ultrasound in a surface layer.
FIG. 6C is a diagram showing the time waveform of the transmitting ultrasound from the surface layer to the focal point.
FIG. 6D is a diagram showing the time waveform of the transmitting ultrasound near the focal point.
FIG. 7 is a diagram showing the transmitting ultrasound from an ultrasound probe.
FIG. 8A is a diagram showing a frequency power spectrum of a transmitting pulse signal of a driving signal of Triad-THI.
FIG. 8B is a diagram showing the frequency power spectrum of the transmitting pulse signal of the driving signal of a low frequency single frequency.
FIG. 9 is a diagram showing a frequency power spectrum of a transmitting pulse signal of a driving signal of triad-THI and the low frequency single frequency.
FIG. 10A is a diagram showing a frequency characteristic of normalization sensitivity of transmitting and receiving by the ultrasound probe.
FIG. 10B is a diagram showing the frequency characteristic of the normalization sensitivity of transmitting by the ultrasound probe.
FIG. 11A is a diagram showing a time characteristic of a signal strength with a driving signal of a first waveform.
FIG. 11B is a diagram showing a power spectrum of the driving signal with the first waveform.
FIG. 12A is a diagram showing the time characteristic of the signal strength of the transmitting ultrasound corresponding to the driving signal with the first waveform.
FIG. 12B is a diagram showing a power spectrum of the transmitting ultrasound corresponding to the driving signal with the first waveform.
FIG. 12C is a diagram showing an envelope curve of an absolute value of the signal strength of the transmitting ultrasound corresponding to the driving signal with the first waveform.
FIG. 12D is a diagram showing the normalization envelope curve of the signal strength of the transmitting ultrasound corresponding to the driving signal with the first waveform.
FIG. 13A is a diagram showing the time characteristic of the signal strength of the driving signal with a second waveform.
FIG. 13B is a diagram showing the power spectrum of the driving signal with the second waveform.
FIG. 14A is a diagram showing the time characteristic of the signal strength of the transmitting ultrasound corresponding to the driving signal with the second waveform.
FIG. 14B is a diagram showing the power spectrum of the transmitting ultrasound corresponding to the driving signal with the second waveform.
FIG. 14C is a diagram showing the normalization envelope curve of the transmitting ultrasound corresponding to the driving signal with the second waveform.
FIG. 15A is a diagram showing the time characteristic of the signal strength of the driving signal with a third waveform.
FIG. 15B is a diagram showing the power spectrum of the driving signal with the third waveform.
FIG. 16A is a diagram showing the time characteristic of the signal strength of the transmitting ultrasound corresponding to the driving signal with the third waveform.
FIG. 16B is a diagram showing a power spectrum of the transmitting ultrasound corresponding to the driving signal with the third waveform.
FIG. 16C is a diagram showing the normalization envelope curve of the transmitting ultrasound corresponding to the driving signal with the third waveform.
FIG. 17A is a diagram showing the time characteristic of the signal strength of the driving signal with a fourth waveform.
FIG. 17B is a diagram showing the power spectrum of the driving signal with the fourth waveform.
FIG. 18A is a diagram showing the time characteristic of the signal strength of the transmitting ultrasound corresponding to the driving signal with the fourth waveform.
FIG. 18B is a diagram showing the power spectrum of the transmitting ultrasound corresponding to the driving signal with the fourth waveform.
FIG. 18C is a diagram showing the normalization envelope curve of the transmitting ultrasound corresponding to the driving signal with the fourth waveform.
FIG. 19A is a diagram showing the time characteristic of the signal strength of the driving signal with a fifth waveform.
FIG. 19B is a diagram showing the power spectrum of the driving signal with the fifth waveform.
FIG. 20A is a diagram showing the time characteristic of the signal strength of the transmitting ultrasound corresponding to the driving signal with the fifth waveform.
FIG. 20B is a diagram showing the power spectrum of the transmitting ultrasound corresponding to the driving signal with the fifth waveform.
FIG. 20C is a diagram showing the normalization envelope curve of the transmitting ultrasound corresponding to the driving signal with the fifth waveform.
FIG. 21A is a diagram showing the time characteristic of the signal strength of the driving signal with a sixth waveform.
FIG. 21B is a diagram showing the power spectrum of the driving signal with the sixth waveform.
FIG. 22A is a diagram showing the time characteristic of the signal strength of the transmitting ultrasound corresponding to the driving signal with the sixth waveform.
FIG. 22B is a diagram showing the power spectrum of the transmitting ultrasound corresponding to the driving signal with the sixth waveform.
FIG. 22C is a diagram showing the normalization envelope curve of the transmitting ultrasound corresponding to the driving signal with the sixth waveform.
FIG. 23 is a schematic diagram showing a power spectrum of transmitting ultrasound transmitted from an inner side element group and a power spectrum of transmitting ultrasound transmitted from an outer side element group.
FIG. 24A is a diagram showing a two-dimensional sound pressure distribution of a first ultrasound beam.
FIG. 24B is a diagram showing the two-dimensional sound pressure distribution of a second ultrasound beam.
FIG. 24C is a diagram showing the two-dimensional sound pressure distribution of a combined ultrasound beam.

### Detailed Description of Embodiments

Hereinafter, one or more embodiments of the present invention will be described with reference to the drawings. However, the scope of the invention is not limited to the disclosed embodiments.

The present invention is described in detail with reference to the drawings. In the description below, the same reference numeral is applied to the components which have the same function and the same configuration and the description is omitted.

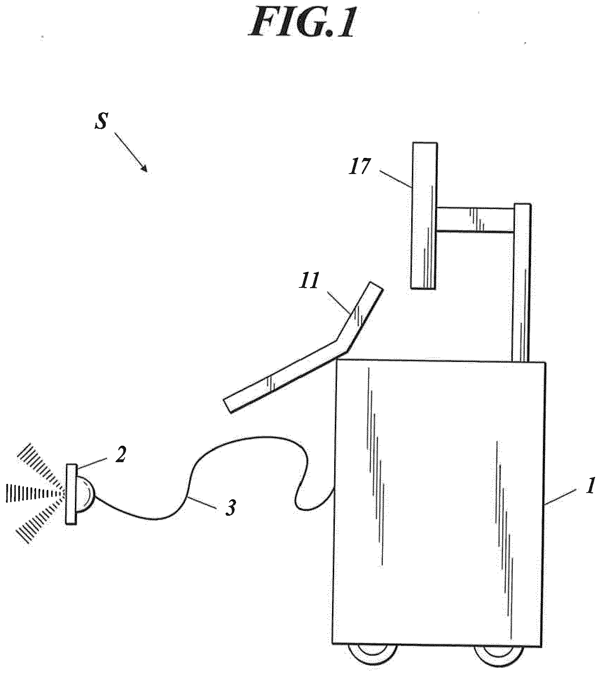

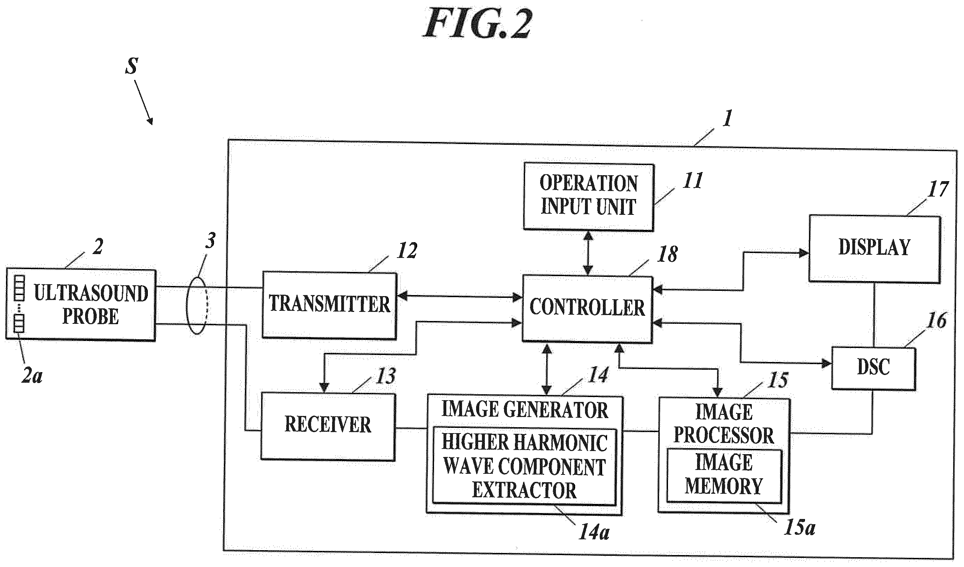

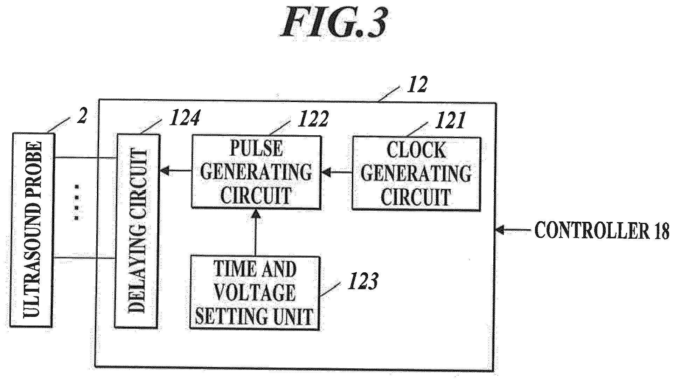

First, the apparatus configuration of the ultrasound diagnostic apparatus S of the present embodiment is described with reference to FIG. 1 to FIG. 3. FIG. 1 is an external diagram of the ultrasound diagnostic apparatus S according to the present embodiment. FIG. 2 is a block diagram showing a functional configuration of the ultrasound diagnostic apparatus S. FIG. 3 is a block diagram showing a functional configuration of a transmitter 12.

As shown in FIG. 1 and FIG. 2, the ultrasound diagnostic apparatus S according to the present embodiment includes an ultrasound diagnostic apparatus main body 1 and an ultrasound probe 2. The ultrasound probe 2 transmits the ultrasound (transmitting ultrasound) to the subject such as a live body (not shown) and receives receiving ultrasound including reflected ultrasound reflected on the subject and scattered ultrasound. The ultrasound diagnostic apparatus main body 1 is connected to the ultrasound probe 2 through a cable 3. The ultrasound diagnostic apparatus main body 1 transmits a driving signal of an electric signal to the ultrasound probe 2 and allows the ultrasound probe 2 to transmit the transmitting ultrasound to the subject. The ultrasound diagnostic apparatus main body 1 images the internal state of the subject as ultrasound image data based on the receiving signal which is an electric signal generated in the ultrasound probe 2 in response to the receiving ultrasound from the subject received by the ultrasound probe 2.

The ultrasound probe 2 includes transducers 2a including a piezoelectric element and an acoustic lens which focuses the transmitting ultrasound toward the focal point. For example, the transducers 2a are aligned in a one-dimensional array in an orientation direction. According to the present embodiment, for example, an ultrasound probe 2 includes 192 transducers 2a. The transducers 2a can be arranged in a two-dimensional array. The number of transducers 2a can be set freely. According to the present embodiment, the ultrasound probe 2 may employ an electronic scanning probe with a linear scanning method. Alternatively, either the electronic scanning method or the mechanical scanning method can be employed. Alternatively, the linear scanning method, the sector scanning method or the convex scanning method can be employed.

As shown in FIG. 2, for example, the ultrasound diagnostic apparatus main body 1 includes an operation input unit 11, a transmitter 12, a receiver 13, an image generator 14, an image processor 15, a DSC (digital scan converter) 16, a display 17, and a controller 18.

For example, the operation input unit 11 includes various switches, buttons, a track ball, a mouse, a keyboard, etc. to input a command to instruct the start of diagnosis or input of data such as individual information of the subject, and the operation signal is output to the controller 18.

The transmitter 12 is a circuit which supplies an electric driving signal to the ultrasound probe 2 through the cable 3, and allows the ultrasound probe 2 to generate the transmitting ultrasound according to control by the controller 18. As the later-described Triad-THI, the transmitter 12 is able to generate a driving signal including components of fundamental waves f1, f2, and f3 of three frequencies. The transmitter 12 is able to generate and output a driving signal with a different driving waveform for each transducer 2a of the ultrasound probe 2. According to the present embodiment, "different driving waveform" means a different driving control signal. Therefore, when the driving control signal is the same but the waveform is different due to only the voltage amplitude in proportion with the power voltage being different, that is, the waveform has the relation of corresponding to the transmitting apodization, such case is not considered to be the "different driving waveform". Specifically, the transmitter 12 divides the transducers 2a of the transmitting opening of the ultrasound probe 2 to two types which are an inner side element group and an outer side element group. The transmitter 12 generates a driving signal for a different driving waveform toward the same focal point, and outputs the above to both the inner side element group and the outer side element group.

For example, as shown in FIG. 3, the transmitter 12 includes, a clock generating circuit 121, a pulse generating circuit 122, a time and voltage setting unit 123, and a delay circuit 124.

The clock generating circuit 121 is a circuit which generates a clock signal which determines the transmitting timing and the transmitting frequency of the driving signal. The pulse generating circuit 122 is a circuit which generates a pulse signal as a driving signal at a predetermined cycle. For example, the pulse generating circuit 122 switches the voltage of 3 values (+HV/0 (GND)/-HV), 5 values (+HV/+MV/0(GND)/-MV/- HV) and outputs the above to generate the driving signal by a rectangular wave. Here, the amplitude of the pulse signal is the same at the positive polarity and the negative polarity but the present invention is not limited to the above. According to the present embodiment, the voltage of 3 values and 5 values are switched to output the driving signal, but the present invention is not limited to 3 values and 5 values, and a suitable number of values can be set. Preferably, the number is 5 values or less. With this, the freedom of control of the frequency components can be enhanced at a low cost, and the transmitting ultrasound with high resolution can be obtained.

The time and voltage setting unit 123 sets for each section the continuing time that the driving signal output from the pulse generating circuit 122 is the same voltage level and the voltage level. That is, the pulse generating circuit 122 outputs the driving signal with the pulse waveform according to the continuing time of each section and the voltage level set by the time and voltage setting unit 123. The continuing time and the voltage level of each section set by the time and voltage setting unit 123 can be varied by input operation by the operation input unit 11.

The delaying circuit 124 is a circuit which sets the delaying time of the transmitting timing of the driving signal for each individual path corresponding to each transducer and delays the transmitting of the driving signal for the set delay time and focuses the transmitting beam including the transmitting ultrasound.

According to the control by the controller 18, the transmitter 12 as described above sequentially switches the transducers 2a to which the driving signal is supplied shifting a predetermined number for each transmission/reception of the ultrasound. The scanning is performed by supplying the driving signal to the transducers 2a selected to output.

According to the above-described embodiment, the pulse inversion method can be performed to extract the later described higher harmonic wave component. Therefore, when the pulse inversion method is performed, the transmitter 12 is able to transmit a first pulse signal and a second pulse signal with the polarity inverted from the first pulse signal on the same scanning line with a time interval in between. Here, the second pulse signal can be transmitted by inverting the polarity of at least one of the plurality of duties of the first pulse signal. The second pulse signal may be inverted timewise from the first pulse signal.

The receiver 13 is a circuit which receives an electric receiving signal from the ultrasound probe 2 through the cable 3 according to the control by the controller 18. The receiver 13 includes, for example, an amplifier, an A/D conversion circuit, and a phase adding circuit. The amplifier is a circuit which amplifies the receiving signal for each individual path corresponding to each transducer 2a at a predetermined amplifying rate. The A/D conversion circuit is a circuit for analog-digital conversion (A/D conversion) of the amplified receiving signal. The phase adding circuit is a circuit which applies delaying time to the A/D converted receiving signal for each individual path corresponding to the transducer 2a to adjust the time phase, and adds the above (phase adding) to generate the sound ray data.

The image generator 14 performs an envelope curve detection process and logarithm amplifying on the sound ray data from the receiver 13 and performs gain adjustment to convert the brightness. With this, B-mode image data is generated. That is, the B-mode image data shows the strength of the receiving signal with brightness. The B-mode image data generated by the image generator 14 is transmitted to the image processor 15. The image generator 14 includes a higher harmonic wave component extractor 14a and generates the B-mode image data from the higher harmonic wave component extracted by the higher harmonic wave component extractor 14a.

The higher harmonic wave component extractor 14a performs the pulse inversion method and extracts the higher harmonic wave component from the receiving signal output from the receiver 13. The even ordered higher harmonic wave component among the higher harmonic wave components (for example, later described 2-order component of 2f1, f1+2, and 0-order component of f2-f1, f3-f2, f3-f1), can be extracted by, according to the pulse inversion method, adding (combining) the receiving signal obtained from the reflected ultrasound to the corresponding two transmitting ultrasound generated from the above-described first pulse signal and second pulse signal, removing the fundamental wave component included in the receiving signal, and then performing the filter process as necessary. The higher harmonic wave component of the odd order higher harmonic wave component (for example, later-described 3f1) can be obtained by subtracting (combining) the same first and second receiving signals, removing the even-order higher harmonic wave component, and then performing the filter process as necessary. The even-order higher harmonic wave component and the odd-order higher harmonic wave component extracted as described above can be added (combined) after performing the phase adjustment process with the all pass filter as necessary, and the higher harmonic wave of the even-order and the odd-order can be combined without canceling to obtain a broadband receiving signal.

The image processor 15 includes an image memory 15a including a semiconductor memory such as a DRAM (Dynamic Random Access Memory). The image processor 15 stores in the image memory 15a the B-mode image data output from the image generator 14 in the unit of frames. The image data in the unit of frames may be called ultrasound image data or frame image data. The image processor 15 suitably reads the ultrasound image data stored in the image memory 15a and outputs the data to the DSC 16.

The DSC 16 performs a process such as a coordinate conversion on the ultrasound image data received by the image processor 15 to convert the data to image signals, and outputs the image signals to the display 17.

Various display apparatuses can be applied as the display 17, for example, LCD (Liquid Crystal Display), CRT (Cathode-Ray Tube) display, organic EL (Electronic Luminescence) display, inorganic EL display, plasma display, and the like. The display 17 displays the ultrasound image on the display screen according to the image signals output from the DSC 16.

For example, the controller 18 includes a CPU (Central Processing Unit), ROM (Read Only Memory), and RAM (Random Access Memory). Various process programs such as a system program stored in the ROM may be read out and deployed in the RAM, and the operation of each unit of the ultrasound diagnostic apparatus S may be centrally controlled according to the deployed program. The ROM includes a nonvolatile memory such as a semiconductor, and stores a system program corresponding to the ultrasound diagnostic apparatus S, various process programs which can be executed on the system program, and various types of data. Such programs may be stored in a format of a computer-readable program code and the CPU sequentially executes the process according to the program code. The RAM forms a work area temporarily storing the various programs executed by the CPU and the data used in such programs.

Specifically, the controller 18 selects the driving signal from the driving signals with different driving waveforms for each transducer 2a of the ultrasound probe 2 (for each later described inner side element group or outer side element group). The controller 18 allows the transmitter 12 to generate the selected driving signal and output the corresponding transducer 2a. The transmitter 12 generates the driving signal selected by the controller 18 and outputs the driving signal to the transducer (transducer group) corresponding to the driving signal.

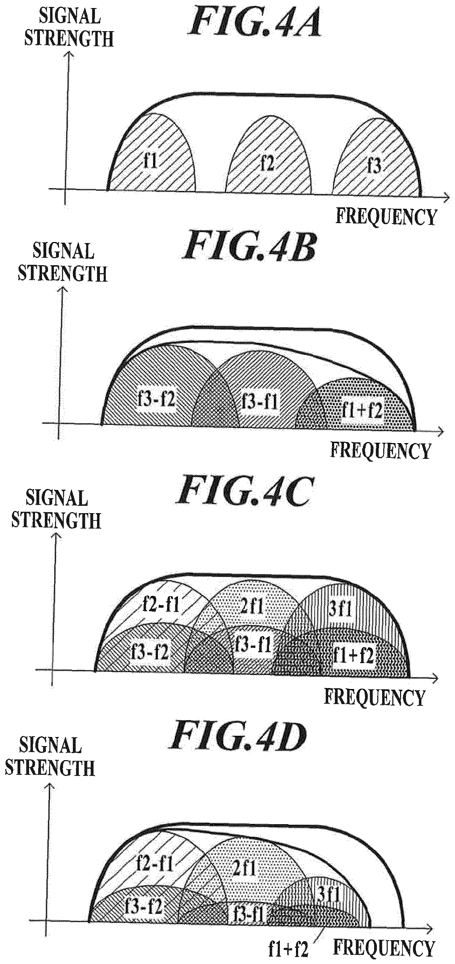

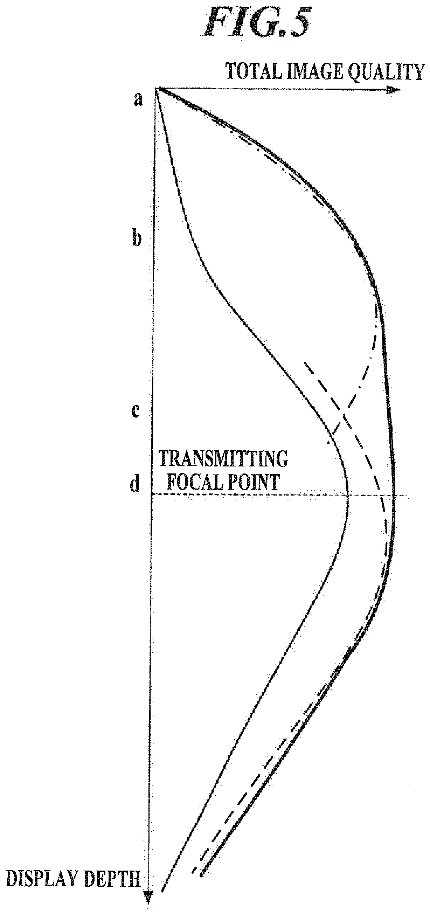

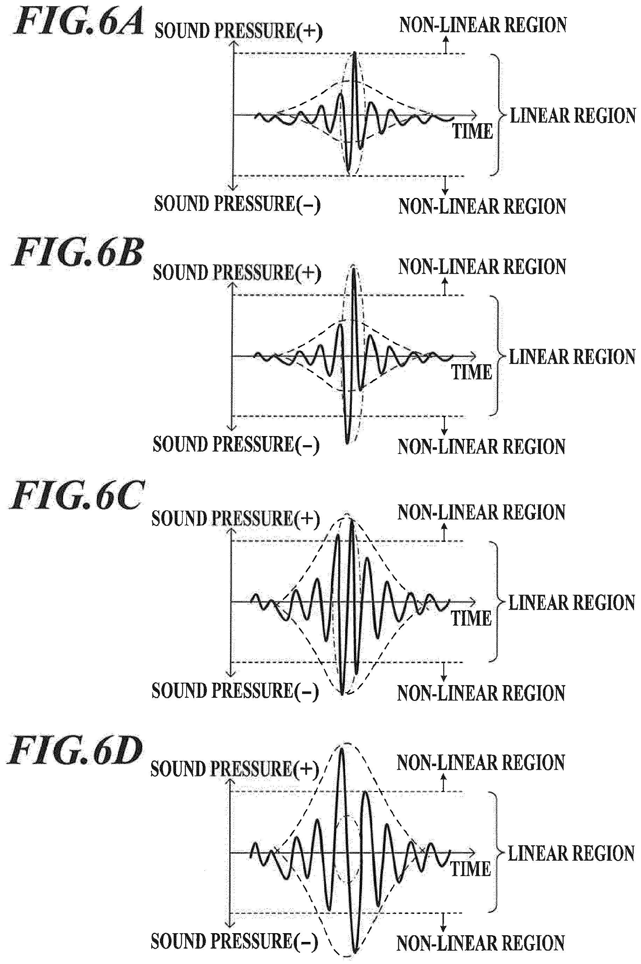

Next, with reference to FIG. 4A to FIG. 6D, the method of generating the driving signal and transmitting/receiving the ultrasound for generating ultrasound image data in Triad-THI according to the present embodiment will be described. FIG. 4A is a diagram showing one example of a frequency characteristic of a signal strength of a transmitting ultrasound according to the present embodiment. FIG. 4B is a diagram showing a frequency characteristic of a signal strength of reflecting ultrasound where a depth is a surface layer. FIG. 4C is a diagram showing a frequency characteristic of a reflecting ultrasound where a depth is near a focal point. FIG. 4D is a diagram showing a frequency characteristic of a signal strength of a reflecting ultrasound where a depth is a portion deeper than a focal point. FIG. 5 is a diagram showing a total image quality for a display depth according to the present embodiment and a conventional example. FIG. 6A is a diagram showing a time waveform of a transmitting ultrasound right after transmitting. FIG. 6B is a diagram showing a time waveform of a transmitting ultrasound in surface layer. FIG. 6C is a diagram showing a time waveform of a transmitting ultrasound from a surface layer to a focal point. FIG. 6D is a diagram showing a time waveform of a transmitting ultrasound near the focal point.

According to the present embodiment, not only is the ultrasound transmission performed in a wide frequency band making full use of the frequency band of the ultrasound probe 2, the waveform of the driving signal and the distribution of the frequency component of the waveform of the transmitting ultrasound is controlled to obtain the higher harmonic wave image with high resolution and S/N in the shallow portion of the subject and to prevent the drastic reduction of resolution in the deep portion. Consequently, a higher harmonic wave image with high uniformity from the shallow portion to the deep portion can be obtained.

More specifically, the transmitting ultrasound includes wide frequency components and the transmitting ultrasound in which the components with high reaching depth abilities as shown in FIG. 6A to FIG. 6D and low frequency components spreading largely timewise and the high frequency components spreading small timewise is transmitted. With this, the higher harmonic wave image is obtained.

The higher harmonic wave component used in THI is generated by the transmitting ultrasound being focused to enhance sound pressure causing propagated non-linearity. When a typical ultrasound probe using an acoustic lens is employed as in the ultrasound probe 2, the focus of the transmitting ultrasound is, focus by refraction of the sound wave by the acoustic lens in the short axis direction, and focus by delay of transmission by electronic focus in the long axis direction. Here, the focusing of the sound wave being different depending on the frequency is used to control generating of the higher harmonic wave.

For example, as shown in FIG. 4A, a configuration which transmits transmitting ultrasound including fundamental waves f1, f2, f3 is considered as Triad-THI. In FIG. 4A, the horizontal axis shows the frequency, the vertical axis shows sensitivity (signal strength) and the bold solid line shows the frequency component of the ultrasound probe (transmitting and receiving frequency band). In FIG. 4B to FIG. 4D, the horizontal axis shows the frequency, the vertical axis shows the signal strength, the normal solid line shows the frequency component collecting each frequency component of the reflecting ultrasound, and the bold solid line shows the frequency component of the ultrasound probe 2 (transmitting and receiving frequency band).

Based on Huygens theory, the focus width of the ultrasound beam is known to be in proportion with the reciprocal of the frequency. According to the configuration of the transmitting ultrasound waveform shown in FIG. 4A, as for the fundamental wave f3 component including a frequency three times the fundamental wave f1, the beam width due to focusing is to be 1/3. That is, the focus is three times in density compared to the fundamental wave f1, and the sound pressure of the region including the component rises easily even with focus by the acoustic lens, and reaches the nonlinear region which generates the higher harmonic wave.

According to FIG. 6A to FIG. 6D, the horizontal axis shows time, the vertical axis shows sound pressure, the broken line shows the time waveform region mainly consisting of the fundamental wave f1, f2 component in the transmitting ultrasound, and the short and long line similarly shows the time waveform region mainly consisting of the fundamental wave f3 component. The region in which the sound pressure is higher than the positive threshold or lower than the negative threshold represents the nonlinear region and the region in which the sound pressure is equal to or less than the positive threshold and equal to or larger than the negative threshold represents the linear region. The display depth a shown in FIG. 5 corresponds to the depth shown in FIG. 6A, the display depth b shown in FIG. 5 corresponds to the depth shown in FIG. 6B, the display depth c shown in FIG. 5 corresponds to the depth shown in FIG. 6C, the display depth d shown in FIG. 5 corresponds to the depth shown in FIG. 6D.

As shown in FIG. 6A, right after transmitting the transmitting ultrasound from the ultrasound probe 2, the fundamental component is within the linear region, but the component with the high frequency is high as shown with the localized short and long line. As shown in FIG. 6B, the transmitting ultrasound of the surface layer portion of the subject (focus by the acoustic lens), the region mainly of the fundamental wave f3 component with high frequency and high focus reaches the nonlinear region by acoustic lens focus, and causing the propogating speed difference, the higher harmonic wave is generated. Therefore, in the surface layer portion as the shallow portion, even if the electronic focus focal point is in the deep portion, the higher harmonic wave component with a broad band with the sound difference of f3 as the center as shown in FIG. 4B is generated by the acoustic lens. Among the transmitting ultrasound, only the region including the high frequency components among the waveforms reaches the nonlinear region by focus. Therefore, the pulse length of the entire waveform is long but the nonlinear region reaching portion which generates the higher harmonic wave is short, and the image with a high resolution can be obtained. As shown in FIG. 4B, as for the higher harmonic component generated, reflected and received from right after transmitting to the surface layer, the component derived from the fundamental wave f3 has a stronger signal strength than the component derived from the fundamental wave f1, f2.

As shown in FIG. 6C, in the intermediate region from the surface layer to the long axis focal point, both the higher harmonic wave at the surface layer portion by the fundamental wave f3 component and the higher harmonic wave near the focal point by the fundamental wave f1, f2 component are generated so as to complement each other.

As shown in FIG. 6D, near the focal point, the time waveform region mainly consisting of the fundamental wave f3 which is the high frequency component reduces due to attenuating and advancing of energy to the higher harmonic wave component and reduces to the sound pressure of the linear region. However, instead, the sound pressure of the component mainly consisting of the fundamental wave f1, f2 with the low frequency held in the linear region at the surface layer portion due to weak attenuation reaches the nonlinear region due to electronic focus, and with this, the higher harmonic wave component is generated. Similar to the surface layer region, although the pulse length of the transmitting ultrasound is long, the region reaching the nonlinear region which generates the higher harmonic wave is partial, and the high definition can be obtained. As shown in FIG. 4C, near the focal point, the newly generated higher harmonic wave component is mainly the components derived from the fundamental wave f1, f2. The higher harmonic wave component derived from the fundamental wave f3 is hardly generated and decreases more than the surface layer portion due to attenuation, and the signal strength relation of the reflected and received higher harmonic wave becomes a relation as shown in the drawings. The 3f1 component shown here, that is, the 3-order higher harmonic wave component is extracted by subtracting calculation and frequency band passing process different from other components which are extracted by adding calculation of pulse inversion, and after phase adjustment as necessary, the result is added to the sound ray signal of other higher harmonic wave components. The phase is adjusted as necessary with the all pass filter and according to the wave overlapping theory, the result is combined with the other higher harmonic wave components and the receiving signal with a broad band can be obtained. As shown in FIG. 4D, the attenuation is stronger than generating the higher harmonic wave in the portion deeper than the focal point, and the higher harmonic wave components which can be reflected and received gradually decreases. However, the higher harmonic component derived from the fundamental wave f1, f2 continues generating the higher harmonic wave to a certain extent and does not decrease drastically, whereas the higher harmonic component derived from the fundamental wave f3 receives only the influence of attenuation, and the percentage in the reflected and received higher harmonic wave component becomes very small.

As shown in FIG. 5, the characteristics of the total image quality corresponding to the display depth when the transmitting ultrasound shown in FIG. 4A is used is higher compared to the characteristics of the total image quality of conventional techniques to obtain the higher harmonic wave image using the fundamental wave on the lower frequency side of the frequency band of the ultrasound probe. The total image quality shows the total image quality including S/N and resolution of the ultrasound image of the generated image data. According to FIG. 5, the broken line shows the generating higher harmonic wave component of the first step by the fundamental wave f3 component, and the long and short line shows the generating higher harmonic wave component of the second step by the fundamental wave f1, f2 component. The frequency characteristics of the total image quality collecting the generating higher harmonic wave component of the first step and the second step according to the present embodiment is shown with a bold solid line, and the frequency characteristics of the total image quality of the conventional example is shown with a normal solid line. Since the frequency of the sound wave transmitted in the conventional example is low, in the shallow portion, the sound pressure does not rise enough and the sufficient higher harmonic wave signal cannot be obtained, and with this, the image quality is not enhanced. By using the difference of the focus characteristics, the higher harmonic wave is generated with a plurality of steps, and it is possible to obtain an ultrasound image with high S/N and high resolution throughout the wide region from the surface layer portion to near the focal point.

The transmitting ultrasound waveform described in FIG. 4A is one example, and the present invention is not limited to the above configuration. Among the frequency components composing the transmitting ultrasound, the components with the high frequency are more concentrated with a narrow spread timewise, and it is necessary that the components with the low frequency are wide spread timewise to generate the multi-step higher harmonic wave.

The first method to generate the transmitting ultrasound waveform is described in Japanese Patent Application Laid-Open Publication No. 2014-168555. The transmitter 12 generates a driving signal. The frequency power spectrum of the transmitting pulse signal of the driving signal is the frequency band included in the -20 dB transmitting frequency band of the ultrasound probe 2. The driving signal has a strength peak in each of the low frequency side and the high frequency side with reference to a central frequency in the -20 dB transmitting frequency band. Further, the strength in the frequency region between the plurality of strength peaks is -20 dB or more with the maximum strength of the strength peak as the reference.

The second method to generate the transmitting ultrasound waveform is described in Japanese Patent Application Laid-Open Publication No. 2015-103842. The transmitter 12 (pulse generating circuit 122) modulates and combines a plurality (here, 3) of time waveform (pulse) of different frequencies, and this is to be the waveform of the driving signal. The method of modulation is a combining method to combine the waveform using the Hanning window in one or a combination of a method which modulates the amplitude of the time waveform of the frequency (amplitude modulation) or a method which transitions the frequency (frequency modulation).

Preferably, when the waveform is combined, a suitable magnification is multiplied to the amplitude of the first to third waveform and combined. Such magnification is determined so that the transmitting sensitivity of the ultrasound probe is corrected and the transmitting ultrasound with the desired frequency strength ratio is transmitted. AM modulation is not limited to the method using the window with the same time width, and the method using the window with the different time width can be used. The number of frequency components used in the combining is not limited. However, in order to structure the transmitting ultrasound waveform, at least two frequencies, preferably three or more frequencies are combined to generate the driving waveform. Among the waveforms used for combining, preferably at least the frequency component two or more times higher than the lowest frequency components is included.

The waveform of the driving signal obtained by combining the plurality of frequency components can be used for driving as is by the transmitter 12 as the arbitrary waveform transmitter, but alternatively, driving can be executed using the approximate driving waveform assigning the state number necessary to drive with the transmitter 12 with the voltage state number limited, for example, 5 value voltage value. That is, the pulse generating circuit 122 generates the driving signal by assigning the continuing time and the voltage level of each section set by the time and voltage setting unit 123 to the combined waveform based on the waveform combining the time waveform modulated by at least one of the AM modulation or the FM modulation, applies the delay of the delay circuit 124 to the driving signal, and outputs the result to the ultrasound probe 2. Alternatively, the waveform information already assigned can be stored in the storage (not shown), and the driving signal can be generated based on such information. The latter does not make the apparatus complicated and is therefore preferable. The driving using an arbitrary waveform transmitter has the merit of being able to finely adjust the control of driving, but in practical use, voltage state number of about 5 values can sufficiently control the driving, and such configuration has the merit of preventing the cost of the apparatus rising.

Further, when FM modulation is performed, it is preferable that the modulation transitions from low frequency to high frequency to low frequency during the time that the waveform continues in order to obtain the transmitting ultrasound waveform. In addition, performing FM modulation on the frequency component lowest among the combined waveform is preferable to increase the low frequency component while maintaining the frequency band width.

The third method to generate the transmitting ultrasound waveform is described in Japanese Patent Application No. 2015-103842. According to this method, a broadband is obtained by scattering the continuing time of one section of the driving signal. The time that one value of the waveform of the time characteristic of the signal strength of the driving signal continues is one section shown with both arrows. The one value here does not consider the voltage fluctuation as the actual value due to variation in the power supply voltage, etc. and the time the one value continues as the logical value of the driving signal is to be the one section. The continuing time of each section of the driving signal obtained as described above is considered to be 0.5 wave and the frequency is converted.

Here, the frequency characteristic of the normalization sensitivity [dB] of transmitting or transmitting and receiving of the ultrasound probe 2 is described. According to the normalization sensitivity, the value of the maximum sensitivity is 0 [dB]. The -6 dB sensitivity band of transmitting and receiving of the ultrasound probe 2 is to be a transmitting/receiving -6 dB band and the -20 dB sensitivity band of transmitting and receiving is to be a transmitting/receiving -20 dB band.

In a waveform which repeats a certain driving time, considering the frequency three order higher harmonic wave component converting value included in the rectangular wave, the frequency component is included only in a portion of the transmitting -20 dB band of the ultrasound probe 2. The output sound wave becomes a narrowband and it is not possible to obtain the broadband higher harmonic wave component. However, by scattering the continuing time of one section and dispersing the regularity of the driving pulse, the driving frequency component can be obtained evenly in the transmitting -20 dB frequency band of the ultrasound probe 2, the band evenness is enhanced, and the higher harmonic wave generated from these components can obtain broadband components. The continuing time of one section is short nearer to the center, and by setting the edge portion longer, the timewise spread of each frequency component in the time waveform is controlled. With this, a multi-step generation of the higher harmonic wave is possible.

However, when the sections are scattered too much, the low frequency component which largely contributes to the deep higher harmonic wave generating relatively decreases, and the penetration which is the desired purpose decreases. Preferably, as for the degree of scattering, each section is considered to be 0.5 wave and the frequency is converted, and a standard deviation of the value normalizing the above at the central frequency of the transmitting/receiving -6 dB band of the ultrasound probe 2 is between 0.1 to 0.3. For example, when the continuing time of the section is 125 [n sec], the frequency conversion value when setting to 0.5 wave is 4 [MHz]. If the central frequency of the transmitting/receiving -6 dB band is 10.25 [MHz], the normalization value is 0.39.

The frequency value when the frequency of each section is converted with 0.5 wave is preferably between 1/3 of the frequency of the lower limit value of the transmitting -20 dB frequency band of the ultrasound probe 2 and the frequency of the upper limit value of the transmitting -20 dB frequency band of the ultrasound probe 2. When the frequency value is lower than 1/3 of the -20 dB frequency band lower limit value of the ultrasound probe 2, not only the one-order component of the frequency but also the three-order higher harmonic wave frequency component inevitably included due to being a rectangular wave becomes lower than the -20 dB frequency band lower limit value frequency and do not contribute to transmitting of the transmitting ultrasound. Similarly, when the frequency value is higher than the upper limit value of the transmitting -20 dB frequency band of the ultrasound probe 2, both the one-order component and the three-order component becomes higher than the transmitting -20 dB frequency band upper limit value and do not contribute to transmitting of the transmitting ultrasound.

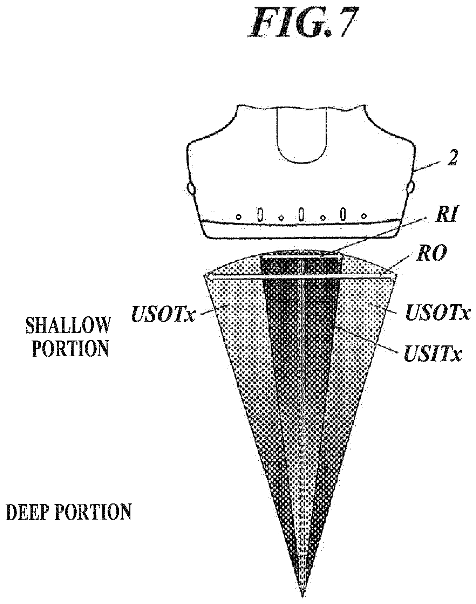

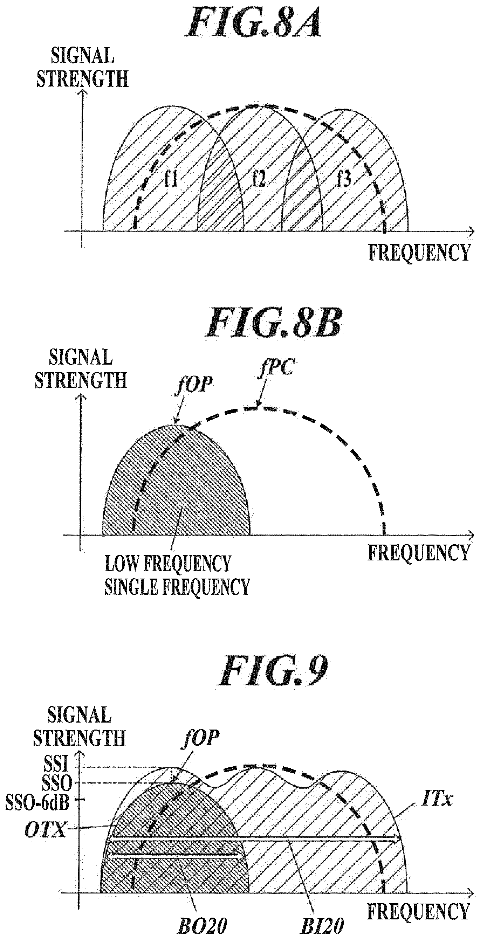

Next, with reference to FIG. 7 to FIG. 9, the output of the transmitting ultrasound corresponding to the driving signal with different driving waveforms input to the ultrasound probe 2 is described. FIG. 7 is a diagram showing the transmitting ultrasound from the ultrasound probe 2. FIG. 8A is a diagram showing the frequency power spectrum of the transmitting pulse signal of the driving signal of Triad-THI. FIG. 8B is a diagram showing the frequency power spectrum of the transmitting pulse signal of the driving signal of the low frequency single frequency. FIG. 9 is a diagram showing the frequency power spectrum of the transmitting pulse signal of the driving signal of the Triad-THI and the low frequency single frequency.

As shown in FIG. 7, according to the present embodiment, among the plurality of transducers 2a of the transmitting opening of the linear scanning type ultrasound probe 2, the driving signal of the Triad-THI is input to the inner side element group which is the plurality of transducers 2a continuously positioned on the inner side in the orientation direction, and the transmitter 12 outputs the transmitting ultrasound USITx of the Triad-THI. In addition to the above, the driving signal with the low frequency single frequency is input to the outer side element group which is the plurality of transducers 2a continuously positioned on the left and right of the inner side element group in the orientation direction, and the transmitter 12 outputs the transmitting ultrasound USOTx of (including component of) the low frequency single frequency. The focal point distance of the transmitting ultrasound USITx of the Triad-THI is equal to the focal point distance of the transmitting ultrasound USOTx of the low frequency single frequency. In FIG. 7, as for the transmitting opening of the ultrasound probe 2, the range of the orientation direction that the transducers 2a of the inner side element group and the outer side element group are arranged is to be the range RO and the range of the orientation direction that the transducers 2a of the inner side element group are arranged is to be the range RI.

As shown in FIG. 8A, the frequency power spectrum of the transmitting pulse signal of the driving signal of the Triad-THI input to the transducer 2a of the inner side element group includes the frequency component of the fundamental waves f1, f2, f3. According to FIG. 8A, the horizontal axis shows the frequency, the vertical axis shows the signal strength (sensitivity) of the driving signal, and the broken line shows the transmitting frequency band of the ultrasound probe 2. The same can be said for FIG. 8B and FIG. 9. As for the fundamental wave f1 which is the low frequency, the transmitting ultrasound does not attenuate in the shallow portion, the penetration is high, the orientation resolution is high, and contributes to enhancing the speckle graininess in the deep portion. As for the fundamental waves f2, f3 with high frequency, the transmitting ultrasound attenuates in the shallow portion, and contributes to enhancing the speckle graininess in the shallow portion and enhancing the S/N in the shallow portion.

As shown in FIG. 8B, the frequency power spectrum of the transmitting pulse signal of the driving signal of the low frequency single frequency input in the transducer 2a of the outer side element group includes the frequency component of the low frequency single frequency. As for the low frequency single frequency, the transmitting ultrasound does not attenuate in the shallow portion, the penetration is high, the orientation resolution is high, and contributes to enhancing the speckle graininess in the deep portion. The driving frequency of the low frequency single frequency does not include the high frequency component, and the surface temperature of the ultrasound probe 2 becomes low. The low frequency single frequency can be the fundamental wave f1. According to FIG. 8B, the central frequency of the -20dB transmitting frequency band (-20 transmitting frequency band) of the normalization sensitivity (sensitivity with the maximum sensitivity being 0 dB) of transmitting ultrasound of the ultrasound probe 2 is to be frequency fPC, and the frequency of the maximum strength peak of the frequency power spectrum of the low frequency single frequency is to be the frequency fOP. As shown in FIG. 8A, the central frequency of the -20 transmitting frequency band of the ultrasound probe 2 is to be the frequency fPC.

The signal component ITx of the transmitting pulse signal of the driving signal of the Triad-THI input in the transducer 2a of the inner side element group shown in FIG. 8A and the signal component OTx of the transmitting pulse signal of the driving signal of the low frequency single frequency output from the transducer 2a of the outer side element group shown in FIG. 8B are overlapped on each other. This is to be the frequency power spectrum of the signal component ITx, OT x of the driving signal to the inner side element group and the outer side element group as shown in FIG. 9.

According to FIG. 9, the -20 dB frequency band when the maximum value (maximum strength) of the signal strength of the signal component ITx in the -20 dB transmitting frequency band of the ultrasound probe 2 is 0 dB is -20 dB frequency band BI20, the-20dB frequency band when the maximum value of the signal strength of the signal component OTx is 0 dB is -20 dB frequency band BO20. According to FIG. 9, in the frequency power spectrum of the transmitting pulse signal of the driving signal of the outer side element group, the signal strength of frequency fOP of the inner side element group with respect to the signal strength in the same frequency fOP showing the maximum signal strength SSO of the frequency band included in the -20 dB transmitting frequency band of the ultrasound probe 2 is to be signal strength SSI. Only the maximum value of the signal strength and the band width within the -20 dB transmitting frequency band of the ultrasound probe as described above is considered, and the maximum value of the signal strength existing outside the frequency band and the band width extending outside the frequency band is not considered.

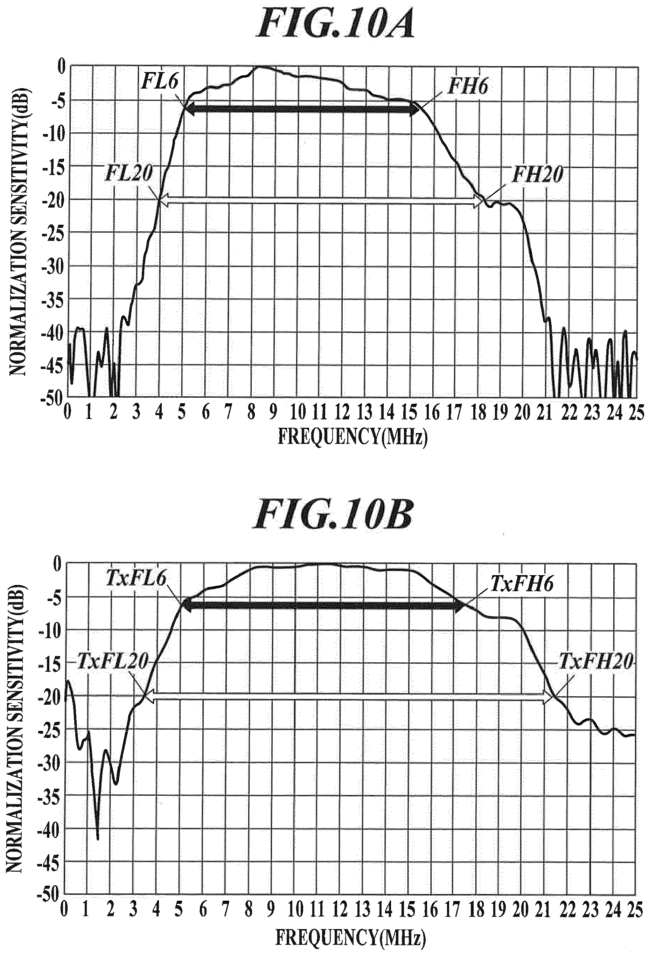

Next, with reference to FIG. 10A to FIG. 23, a specific example of the ultrasound probe 2, and the embodiments as examples of the transmitting conditions such as the driving signal, the transmitting ultrasound, etc. and the comparative examples are described.

When the driving signal and the transmitting ultrasound is generated in the examples 1 to 10 and the comparative examples 1 to 5, the ultrasound probe 2 including the transmitting and receiving characteristics and the transmitting characteristics shown in FIG. 10A and FIG. 10B is used. FIG. 10A is a diagram showing frequency characteristics of the normalization sensitivity of transmitting and receiving with the ultrasound probe 2. FIG. 10B is a diagram showing frequency characteristics of the normalization sensitivity of transmitting with the ultrasound probe 2.

Here, the frequency characteristics of the ultrasound probe 2 is described with reference to FIG. 10A and FIG. 10B. According to FIG. 10A and FIG. 10B, the horizontal axis shows the frequency [MHz] and the vertical axis shows the normalization sensitivity [dB]. According to the normalization sensitivity, the maximum sensitivity value is 0 [dB]. According to FIG. 10A, the -6 dB transmitting /receiving frequency band showing the -6 dB sensitivity band of transmitting and receiving is shown with a black two-direction arrow, the -20 dB transmitting/receiving frequency band showing the -20 dB sensitivity band of transmitting and receiving is shown with a white two-direction arrow. The upper limit value of the -6 dB transmitting/receiving frequency band is frequency FH6, and the lower limit value is frequency FL6. The upper limit value of the -20 dB transmitting/receiving frequency band is frequency FH20 and the lower limit value is frequency FL20.

According to FIG. 10B, the -6 dB transmitting frequency band showing the -6 dB sensitivity band of transmitting is shown with a black two-direction arrow, and the -20 dB transmitting frequency band showing the -20 dB sensitivity band of transmitting is shown with a white two-direction arrow. The upper limit value of the -6 dB transmitting frequency band is to be frequency TxFH6, and the lower limit value is to be frequency TxFL6. The upper limit value of the -20 dB transmitting frequency band is to be frequency TxFH20, and the lower limit value is to be frequency TxFL20. As shown in FIG. 10B, the -20 dB transmitting frequency band is 3.4 to 21.3 [MHz], and the central frequency fPC is 12.35 [MHz].

According to the examples 1 to 10 and the comparative examples 1 to 5, the pulse inversion method using a first pulse signal and a second pulse signal as a driving signal in which the second pulse signal waveform is a waveform inverting the polarity of the first pulse signal waveform is performed in all of the transducers 2a of the transmitting opening of the ultrasound probe 2.

### <Comparative Example 1>

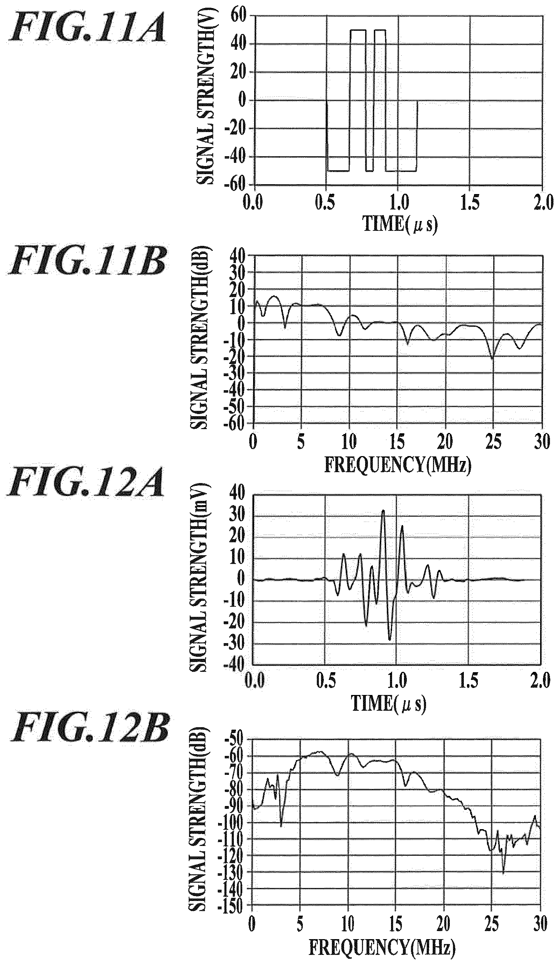

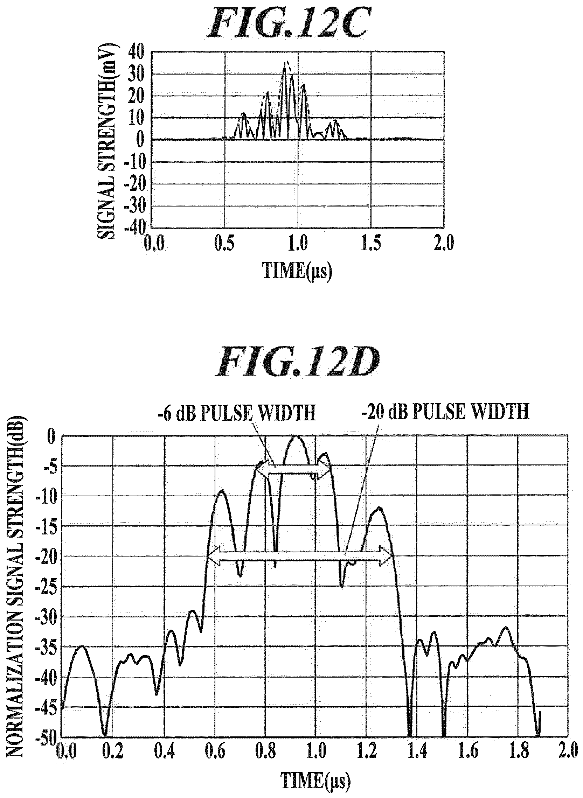

The comparative example 1 is an example in which the transmitting apodization is performed in the driving signal with the same driving waveform without dividing the transducers 2a of the transmitting opening in the ultrasound probe 2 between the outer side element group and the inner side element group, and such driving signal is input in each transducer 2a of the transmitting opening. The waveform (driving waveform) of the driving signal in the comparative example 1 is to be waveform 1. FIG. 11A is a diagram showing a time characteristic of the signal strength of the driving signal with the waveform 1. FIG. 11B is a diagram showing the power spectrum of the driving signal with the waveform 1. FIG. 12A is a diagram showing the time characteristic of the signal strength of the transmitting ultrasound corresponding to the driving signal of waveform 1. FIG. 12B is a diagram showing the power spectrum of the transmitting ultrasound corresponding to the driving signal with the waveform 1. FIG. 12C is a diagram showing the envelope curve of the absolute value of the signal strength of the transmitting ultrasound corresponding to the driving signal with the waveform 1. FIG. 12D is a diagram showing the normalization envelope curve of the signal strength of the transmitting ultrasound corresponding to the driving signal with the waveform 1.

The comparative example 1 performs transmitting apodization which adjusts the amplitude using the Hanning window function and the driving signal with a waveform 1 and different amplitude is input in each transducer 2a of the transmitting opening of the ultrasound probe 2. The channel number (transducer 2a number) of the transmitting opening of the ultrasound probe 2 is 50 as one example, and this is similarly set in the comparative examples 2 to 5, and examples 1 to 10.

The waveform of the driving signal of the Triad-THI of the waveform 1 generated by the transmitter 12 is a waveform of the time characteristic of the signal strength shown in FIG. 11A. The (frequency) power spectrum obtained by Fourier conversion of the driving signal shown in FIG. 11A is to be the frequency characteristic of the signal strength shown in FIG. 11B. The waveform of the transmitting ultrasound transmitted when the driving signal shown in FIG. 11A is input to the ultrasound probe 2 is to be the waveform of the time characteristic of the signal strength shown in FIG. 12A. The (frequency) power spectrum obtained by Fourier conversion of the transmitting ultrasound shown in FIG. 12A is to be the frequency characteristic of the signal strength show in FIG. 12B.

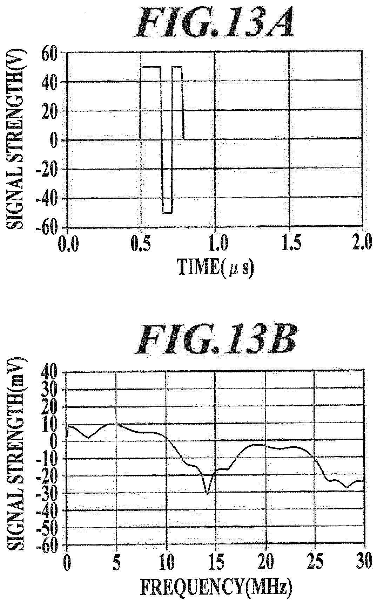

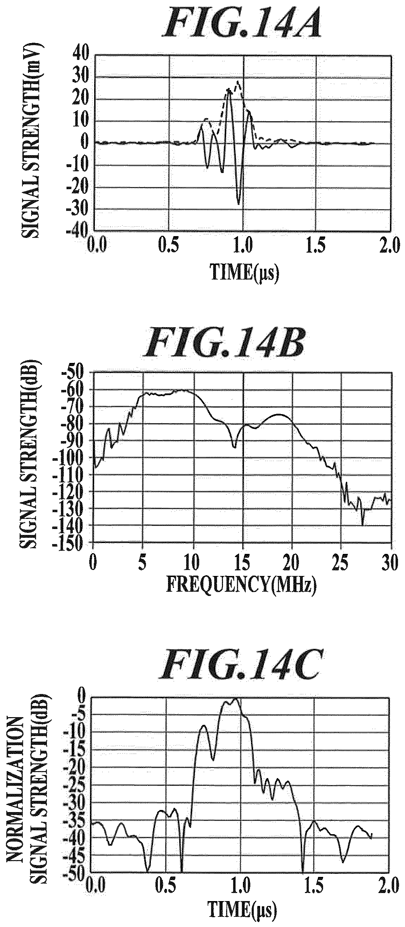

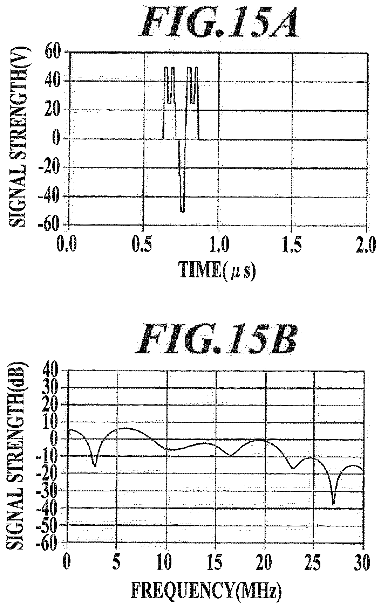

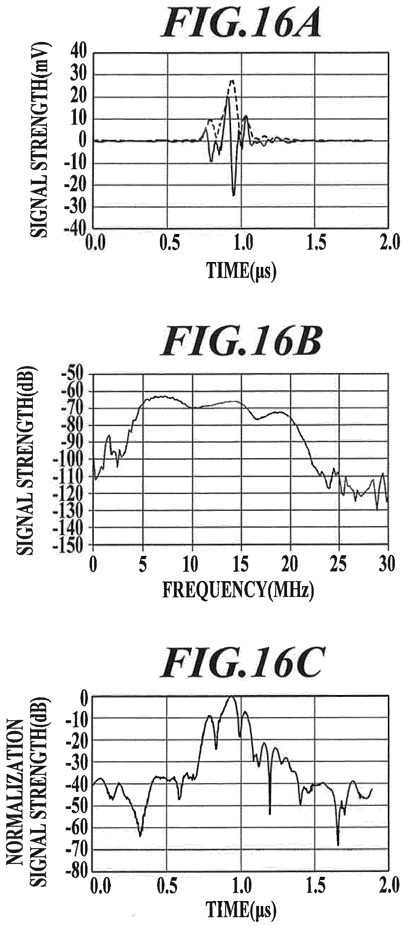

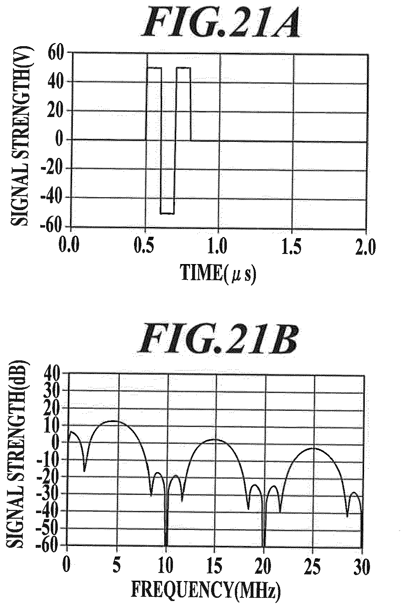

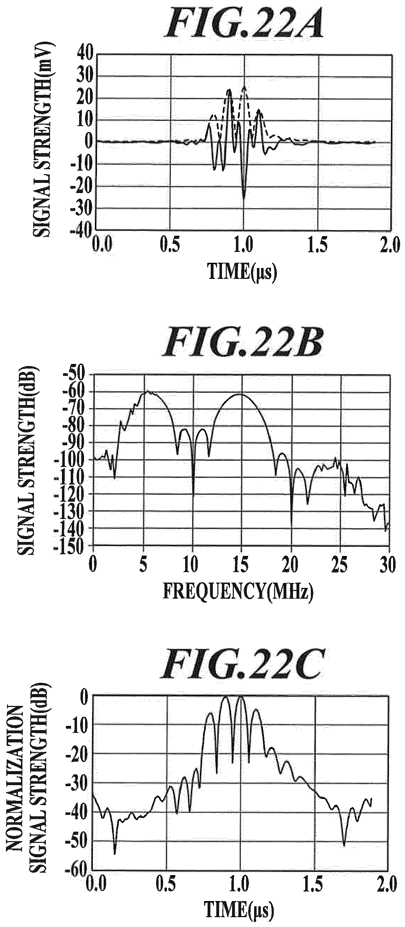

Here, according to FIG. 11A, the horizontal axis shows time [µs], the vertical axis shows the signal strength (voltage) [V], and the same can be said for FIG. 13A, FIG. 15A, ... FIG. 21A. According to FIG. 11B, the horizontal axis shows the frequency [MHz], the vertical axis shows the signal strength [dB], and the same can be said for FIG. 12B ... FIG. 22B. According to FIG. 12A, the horizontal axis shows time [µs], the vertical axis shows the signal strength (voltage) [mV], and the same can be said for FIG. 14A, FIG. 16A, ... FIG. 22A, and FIG. 12C. According to FIG. 12D, the horizontal axis shows the time [µs], the vertical axis shows the normalization signal strength [dB], and the same can be said for FIG. 14C, FIG. 16C, ... FIG. 22C.

Here, with reference to FIG. 12C, FIG. 12D, the calculating method of the pulse width ratio of the transmitting ultrasound corresponding to the driving signal with the waveform 1 shown in FIG. 12A is described. FIG. 12C is a diagram showing the envelope curve of the absolute value of the signal strength of the transmitting ultrasound corresponding to the driving signal with the waveform 1. FIG. 12D is a diagram showing a normalization envelope curve of the signal strength of the transmitting ultrasound corresponding to the driving signal with the waveform 1.

First, the absolute value of the signal strength of the transmitting ultrasound corresponding to the driving signal with the waveform 1 shown in FIG. 12A is described as shown with a solid line in FIG. 12C, and the envelope curve which is the outer edge of the absolute value is described as shown with a broken line in FIG. 12C. The maximum value of the envelope curve of the signal strength of the transmitting ultrasound as shown in FIG. 12A obtained as described above is to be 0 dB, and as shown with the solid line in FIG. 12D, the normalization envelope curve of the signal strength of the transmitting ultrasound shown in FIG. 12A is obtained. Then, in the normalization envelope curve shown in FIG. 12D, the time from the earliest intersection point to the latest intersection point reaching -6 dB and -20 dB is to be -6 dB pulse width and -20 dB pulse width. By dividing the -6 dB pulse width with the -20 dB pulse width, -6 dB/-20 dB pulse width ratio (ratio of -6 dB pulse width with respect to -20 dB pulse width) is calculated.

The -6 dB pulse width of the transmitting ultrasound corresponding to the driving signal with the waveform 1 is 301 [ns], and the -20 dB pulse width of the transmitting ultrasound corresponding to the driving signal with the waveform 1 is 735 [ns], and the -6 dB/ -20 dB pulse width ratio of the transmitting ultrasound corresponding to the driving signal with the waveform 1 is 0.41.

### <Comparative Example 2>

The comparative example 2 is similar to the comparative example 1, but is the example which does not perform the transmitting apodization. The driving waveform of the driving signal of the comparative example 2 is the waveform 1. The transmitting apodization is not performed in the comparative examples 3 to 5, and the examples 1 to 10.

### <Comparative Example 3>

The comparative example 3 inputs the driving signal with the same driving waveform (waveform 1) in the inner side element group and the outer side element group of the transmitting opening of the ultrasound probe 2. As shown in Japanese Patent Application Laid-Open Publication No. 2013-158626, this is an example in which the focal point distance (transmitting focal point) of the inner side element group of the transmitting opening of the ultrasound probe 2 and the transmitting focal point of the outer side element group are different (the transmitting focal point of the inner side element group : 20 [mm] < transmitting focal point of the outer side element group : 35 [mm]). According to the examples 1 to 10 and the comparative examples 1, 2, 4, and 5, the transmitting focal point of the driving signal to all transducers 2a of the transmitting opening in the ultrasound probe 2 is to be equal (focuses to the same focal point).

### <Example 1>

The example 1 inputs the driving signal with the waveform 1 in the inner side element group of the transmitting opening of the ultrasound probe 1, and inputs the driving signal with the waveform 2 in the outer side element group. The waveform 2 is a low frequency single frequency and has a different waveform from the waveform 1. FIG. 13A is a diagram showing the time characteristic of the signal strength of the driving signal with the waveform 2. FIG. 13B is a diagram showing the power spectrum of the driving signal with the waveform 2. FIG. 14A is a diagram showing the time characteristic of the signal strength of the transmitting ultrasound corresponding to the driving signal of the waveform 2. FIG. 14B is a diagram showing the power spectrum of the transmitting ultrasound corresponding to the driving signal of the waveform 2. FIG. 14C is a diagram showing the normalization envelope curve of the transmitting ultrasound corresponding to the driving signal of the waveform 2.

The waveform of the driving signal of the low frequency single frequency of the waveform 2 generated by the transmitter 12 is the waveform of the time characteristic of the signal strength shown in FIG. 13A. The (frequency) power spectrum obtained by Fourier conversion of the driving signal shown in FIG. 13A is the frequency characteristic of the signal strength shown in FIG. 13B. The waveform of the transmitting ultrasound transmitted when the driving signal shown in FIG. 13A is input to the ultrasound probe 2 is the waveform of the time characteristic of the signal strength shown with a solid line in FIG. 14A. The (frequency) power spectrum obtained by performing Fourier conversion on the transmitting ultrasound shown in FIG. 14A is to be the frequency characteristic of the signal strength shown in FIG. 14B.

The envelope curve of the absolute value of the signal strength of the transmitting ultrasound corresponding to the driving signal with the waveform 2 shown in FIG. 14A is obtained as shown with the broken line in the same diagram. Then, as shown in FIG. 14C, the normalization envelope curve of the signal strength of the transmitting ultrasound corresponding to the driving signal with the waveform 2 is obtained. The -6 dB pulse width of the transmitting ultrasound corresponding to the driving signal with the waveform 2 is 186 [ns], and the -20 dB pulse width of the transmitting ultrasound corresponding to the driving signal with the waveform 2 is 400 [ns], and the -6 dB/-20dB pulse width ratio of the transmitting ultrasound corresponding to the driving signal with the waveform 2 is 0.47.

According to the example 1, the percentage of the channel number of the inner side element group (channel number of range RI shown in FIG. 7) with respect to the channel number of the entire transmitting opening of the ultrasound probe 2 (channel number of range RO shown in FIG. 7) is to be 60% (larger than 1/2).

### <Example 2>

The example 2 is an example using the driving signal of the driving waveform similar to the example 1. According to example 2, the percentage of the channel number of the inner side element group with respect to the channel number of the entire transmitting opening of the ultrasound probe 2 is to be 48% (1/16 to 1/2).

### <Example 3>

The example 3 is an example using the driving signal with the driving waveform similar to the example 1. According to example 3, the percentage of the channel number of the inner side element group with respect to the channel number of the entire transmitting opening of the ultrasound probe 2 is to be 24% (1/16 to 1/2).

### <Example 4>

The example 4 is an example using the driving signal of the driving waveform similar to the example 1. According to example 4, the percentage of the channel number of the inner side element group with respect to the channel number of the entire transmitting opening of the ultrasound probe 2 is to be 12% (1/16 to 1/2).

### <Example 5>

The example 5 is an example using the driving signal of the driving waveform similar to the example 1. According to example 5, the percentage of the channel number of the inner side element group with respect to the channel number of the entire transmitting opening of the ultrasound probe 2 is to be 8% (1/16 to 1/2).

### <Example 6>

The example 6 is an example using the driving signal of the driving waveform similar to the example 1. According to example 6, the percentage of the channel number of the inner side element group with respect to the channel number of the entire transmitting opening of the ultrasound probe 2 is to be 4% (smaller than 1/16).

### <Comparative Example 4>

The comparative example 4 is an example where the driving signal in which the driving waveform is the waveform 2 is input to the outer side element group (channel number =50) of the transmitting opening of the ultrasound probe 2 and there is no inner side element group.

### <Comparative Example 5>

The comparative example 5 is an example where the driving signal in which the driving waveform is a waveform 3 (waveform number = 3) is input to the inner side element group (channel number =50) of the transmitting opening of the ultrasound probe 2 and there is no outer side element group. FIG. 15A is a diagram showing the time characteristic of the signal strength of the driving signal with the waveform 3. FIG. 15B is a diagram showing the power spectrum of the driving signal with the waveform 3. FIG. 16A is a diagram showing the time characteristic of the signal strength of the transmitting ultrasound corresponding to the driving signal with the waveform 3. FIG. 16B is a diagram showing the power spectrum of the transmitting ultrasound corresponding to the driving signal with the waveform 3. FIG. 16C is a diagram showing the normalization envelope curve of the transmitting ultrasound corresponding to the driving signal with the waveform 3.

The waveform of the driving signal of the Triad-THI with the waveform 3 generated by the transmitter 12 is the waveform of the time characteristic of the signal strength shown in FIG. 15A. The (frequency) power spectrum obtained by performing the Fourier conversion on the driving signal shown in FIG. 15A is to be the frequency characteristic of the signal strength shown in FIG. 15B. The waveform of the transmitting ultrasound transmitted when the driving signal shown in FIG. 15A is input to the ultrasound probe 2 is the waveform of the time characteristic of the signal strength shown with a solid line in FIG. 16A. The (frequency) power spectrum obtained by performing the Fourier conversion on the transmitting ultrasound shown in FIG. 16A is to be the frequency characteristic of the signal strength shown in FIG. 16B.

The envelope curve of the absolute value of the signal strength of the transmitting ultrasound shown with the solid line corresponding to the driving signal of the waveform 3 shown in FIG. 16A is obtained as shown with the broken line in the same diagram. As shown in FIG. 16C, the normalization envelope curve of the signal strength of the transmitting ultrasound corresponding to the driving signal with the waveform 3 is obtained. The -6 dB pulse width of the transmitting ultrasound corresponding to the driving signal with the waveform 3 is 87 [ns], the -20 dB pulse width of the transmitting ultrasound corresponding to the driving signal with the waveform 3 is 334 [ns], and the -6 dB/ -20 dB pulse width ratio of the transmitting ultrasound corresponding to the driving signal of the waveform 3 is 0.26.

### <Example 7>

The example 7 is an example in which the driving signal with the waveform 3 is input in the inner side element group of the transmitting opening in the ultrasound probe 2, and the driving signal with the waveform 2 is input in the outer side element group. The driving signal with the waveform 2 has the low frequency single frequency and has a different driving waveform from the waveform 3.

### <Example 8>

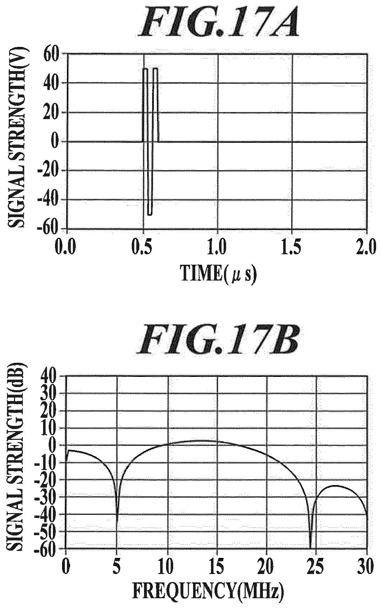

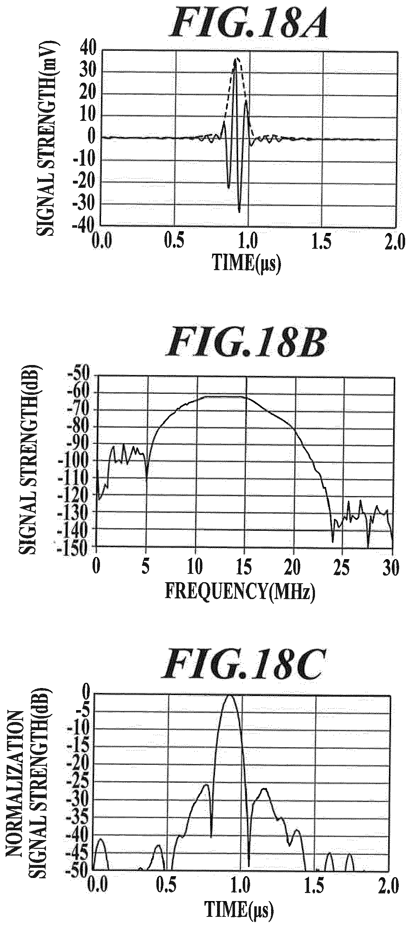

The example 8 is an example in which the driving signal with the waveform 3 is input in the inner side element group of the transmitting opening in the ultrasound probe 2 and the driving signal with a waveform 4 is input in the outer side element group. The waveform 4 is single frequency but not a low frequency and has a waveform different from the waveform 3. FIG. 17A is a diagram showing the time characteristic of the signal strength of the driving signal with the waveform 4. FIG. 17B is a diagram showing the power spectrum of the driving signal with the waveform 4. FIG. 18A is a diagram showing the time characteristic of the signal strength of the transmitting ultrasound corresponding to the driving signal with the waveform 4. FIG. 18B is a diagram showing the power spectrum of the transmitting ultrasound corresponding to the driving signal with the waveform 4. FIG. 18C is the diagram showing the normalization envelope curve of the transmitting ultrasound corresponding to the driving signal with the waveform 4.

The waveform of the single frequency driving signal with the waveform 4 generated by the transmitter 12 has a waveform of the time characteristic of the signal strength as shown in FIG. 17A. The (frequency) power spectrum obtained by Fourier conversion of the driving signal shown in FIG. 17A is to be the frequency characteristic of the signal strength shown in FIG. 17B. The waveform of the transmitting ultrasound transmitted by inputting the driving signal shown in FIG. 17A to the ultrasound probe 2 is to be the waveform of the time characteristic of the signal strength shown with a solid line in FIG. 18A. The (frequency) power spectrum obtained by Fourier conversion of the transmitting ultrasound shown in FIG. 18A is to be the frequency characteristic of the signal strength shown in FIG. 18B.

The envelope curve of the absolute value of the signal strength of the transmitting ultrasound shown with the solid line corresponding to the driving signal with the waveform 4 shown in FIG. 18A is obtained as shown with the broken line in the diagram. Then, as shown in FIG. 18C, the normalization envelope curve of the signal strength of the transmitting ultrasound corresponding to the driving signal with the waveform 4 is obtained. The -6 dB pulse width of the transmitting ultrasound corresponding to the driving signal with the waveform 4 is 119 [ns], the -20 dB pulse width of the transmitting ultrasound corresponding to the driving signal with the waveform 4 is 200 [ns], and the -6 dB/- 20 dB pulse width ratio of the transmitting ultrasound corresponding to the driving signal with the waveform 4 is 0.59.

### <Example 9>

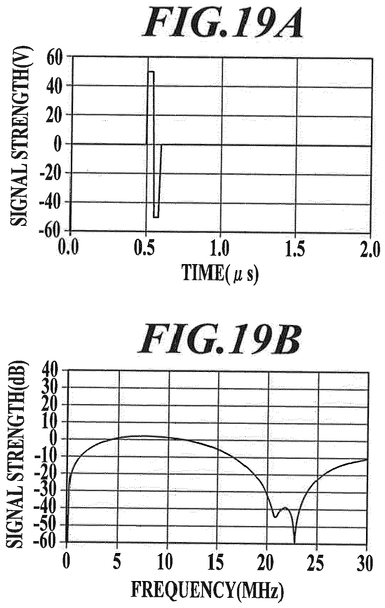

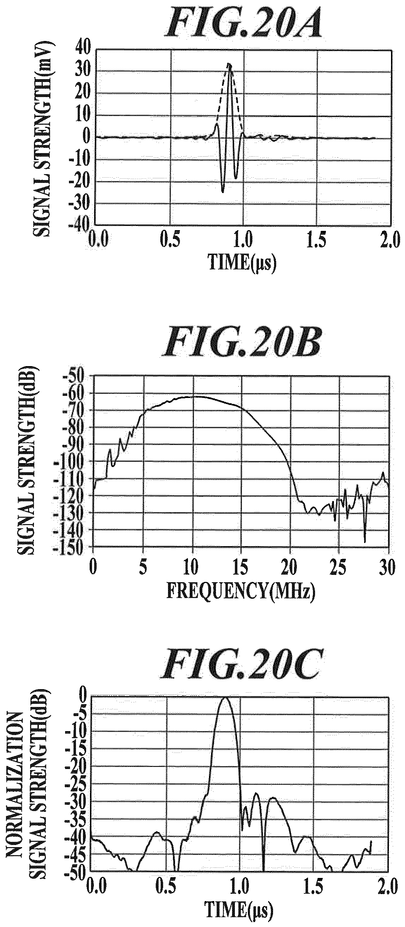

The example 9 is an example in which the driving signal with a waveform 5 which has a driving waveform different from the waveform 2 is input in the inner side element group of the transmitting opening of the ultrasound probe 2, and the driving signal with the waveform 2 is input in the outer side element group. FIG. 19A is a diagram showing the time characteristic of the signal strength of the driving signal with the waveform 5. FIG. 19B is a diagram showing the power spectrum of the driving signal with the waveform 5. FIG. 20A is a diagram showing the time characteristic of the signal strength of the transmitting ultrasound corresponding to the driving signal with the waveform 5. FIG. 20B is the diagram showing the power spectrum of the transmitting ultrasound corresponding to the driving signal with the waveform 5. FIG. 20C is a diagram showing the standardization envelope curve of the transmitting ultrasound corresponding to the driving signal with the waveform 5.

The waveform of the driving signal which is not the Triad-THI, in which the low frequency component has a low signal strength, which has the waveform 5 and which is generated by the transmitter 12 has the waveform of the time characteristic of the signal strength shown in FIG. 19A. The (frequency) power spectrum obtained by performing Fourier conversion on the driving signal shown in FIG. 19A is to be the frequency characteristic of the signal strength shown in FIG. 19B. The waveform of the transmitting ultrasound transmitted by inputting the driving signal shown in FIG. 19A in the ultrasound probe 2 is to be the waveform of the time characteristic of the signal strength shown with a solid line in FIG. 20A. The (frequency) power spectrum obtained by performing Fourier conversion on the transmitting ultrasound shown in FIG. 20A is to be the frequency characteristic of the signal strength shown in FIG. 20B.

The envelope curve of the absolute value of the signal strength of the transmitting ultrasound shown with the solid line corresponding to the driving signal with the waveform 5 shown in FIG. 20A is obtained as shown with the broken line in the diagram. Then, as shown in FIG. 20C, the normalization envelope curve of the signal strength of the transmitting ultrasound corresponding to the driving signal with the waveform 5 is obtained. The -6 dB pulse width of the transmitting ultrasound corresponding to the driving signal with the waveform 5 is 114 [ns], the - 20 dB pulse width of the transmitting ultrasound corresponding to the driving signal with the waveform 5 is 194 [ns], and the -6 dB/- 20dB pulse width ratio of the transmitting ultrasound corresponding to the driving signal with the waveform 5 is 0.59.

### <Example 10>

The example 10 is an example in which the driving signal with the waveform 3 is input in the inner side element group of the transmitting opening in the ultrasound probe 2, and the driving signal with a waveform 6 which has a driving waveform different from the waveform 3 is input in the outer side element group. FIG. 21A is a diagram showing the time characteristic of the signal strength of the driving signal with the waveform 6. FIG. 21B is a diagram showing the power spectrum of the driving signal with the waveform 6. FIG. 22A is a diagram showing the time characteristic of the signal strength of the transmitting ultrasound corresponding to the driving signal with the waveform 6. FIG. 22B is a diagram showing the power spectrum of the transmitting ultrasound corresponding to the driving signal with the waveform 6. FIG. 22C is a diagram showing the normalization envelope curve of the transmitting ultrasound corresponding to the driving signal with the waveform 6.

The waveform of the low frequency single frequency driving signal with the waveform 6 generated by the transmitter 12 is a waveform of the time characteristic of the signal strength shown in FIG. 21A. The (frequency) power spectrum obtained by performing Fourier conversion on the driving signal shown in FIG. 21A is to be the frequency characteristic of the signal strength shown in FIG. 21B. The waveform of the transmitting ultrasound transmitted by inputting the driving signal shown in FIG. 21A in the ultrasound probe 2 is to be the waveform of the time characteristic of the signal strength shown with a solid line in FIG. 22A. The (frequency) power spectrum obtained by performing the Fourier conversion on the transmitting ultrasound shown in FIG. 22A is to be the frequency characteristic of the signal strength shown in FIG. 22B.

The envelope curve of the absolute value of the signal strength of the transmitting ultrasound shown with the solid line corresponding to the driving signal with the waveform 6 shown in FIG. 22A is obtained as shown with the broken line in the diagram. Then, as shown in FIG. 22C, the normalization envelope curve of the signal strength of the transmitting ultrasound corresponding to the driving signal of the waveform 6 is obtained. The -6 dB pulse width of the transmitting ultrasound corresponding to the driving signal with the waveform 6 is 321 [ns], the -20 dB pulse width of the transmitting ultrasound corresponding to the driving signal with the waveform 6 is 496 [ns], and the -6 dB/-20 dB pulse width ratio of the transmitting ultrasound corresponding to the driving signal with the waveform 6 is 0.65.

### <Image Evaluation>

Each item of the transmitting condition regarding the driving signal and the transmitting ultrasound in the examples 1 to 10 and comparative examples 1 to 5 and the image evaluation result of the ultrasound image of the image data generated using the driving signal and the transmitting ultrasound in the ultrasound diagnostic apparatus S is shown in the following table I, table II, and table III.

**TABLE I**

| | TRANSMITTING CONDITION | | | | | |
|---|---|---|---|---|---|---|
| | TRANSMITTING OPENING | | TRANSMITTING FOCAL POINT (mm) | DRIVING WAVEFORM | -20 dB BAND WIDTH OF DRIVING SIGNAL IN -20 dB TRANSMITTING FREQUENCY BAND OF ULTRASOUND PROBE | |
| | INNER SIDE CHANNEL (%) | OUTER SIDE CHANNEL (%) | | | | |
| | | | | | INNER SIDE DRIVING SIGNAL UPPER LIMIT/ LOWER LIMIT FREQUENCY (MHz) | OUTER SIDE DRIVING SIGNAL UPPER LIMIT/ LOWER LIMIT FREQUENCY (MHz) |
| COMPARATIVE EXAMPLE 1 | 50 (100) | - | 35 | WAVEFORM 1 | 3.4/16.0 | - |
| COMPARATIVE EXAMPLE 2 | | | | | | |
| COMPARATIVE EXAMPLE 3 | 24 (48) | 26 (52) | INNER SIDE=20 OUTER SIDE=35 | | | |
| EXAMPLE 1 | 30 (60) | 20 (40) | | | | 3.4/11.5 |
| EXAMPLE 2 | 24 (48) | 26 (52) | | | | |
| EXAMPLE 3 | 12 (24) | 38 (76) | | INNER SIDE =WAVEFORM 1 | | |
| EXAMPLE 4 | 6 (12) | 44 (88) | | OUTER SIDE =WAVEFORM 2 | | |
| EXAMPLE 5 | 4 (8) | 46 (92) | | | | |
| EXAMPLE 6 | 2 (4) | 48 (96) | | | | |
| COMPARATIVE EXAMPLE 4 | - | 50 (100) | | WAVEFORM 2 | - | |
| COMPARATIVE EXAMPLE 5 | 50 (100) | - | 35 | WAVEFORM 3 | 3.4/21.3 | - |
| EXAMPLE 7 | 6 (12) | 44 (88) | | INNER SIDE =WAVEFORM 3 | | 3.4/11.5 |
| | | | | OUTER SIDE =WAVEFORM 2 | | |
| EXAMPLE 8 | | | | INNER SIDE =WAVEFORM 3 | | 5.5/21.3 |
| | | | | OUTER SIDE =WAVEFORM 4 | | |
| EXAMPLE 9 | | | | INNER SIDE =WAVEFORM 5 | 3.4/18.6 | 3.4/11.5 |
| | | | | OUTER SIDE =WAVEFORM 2 | | |
| EXAMPLE 10 | | | | INNER SIDE =WAVEFORM 3 | 3.4/21.3 | 3.4/7.7 |
| | | | | OUTER SIDE =WAVEFORM 6 | | |

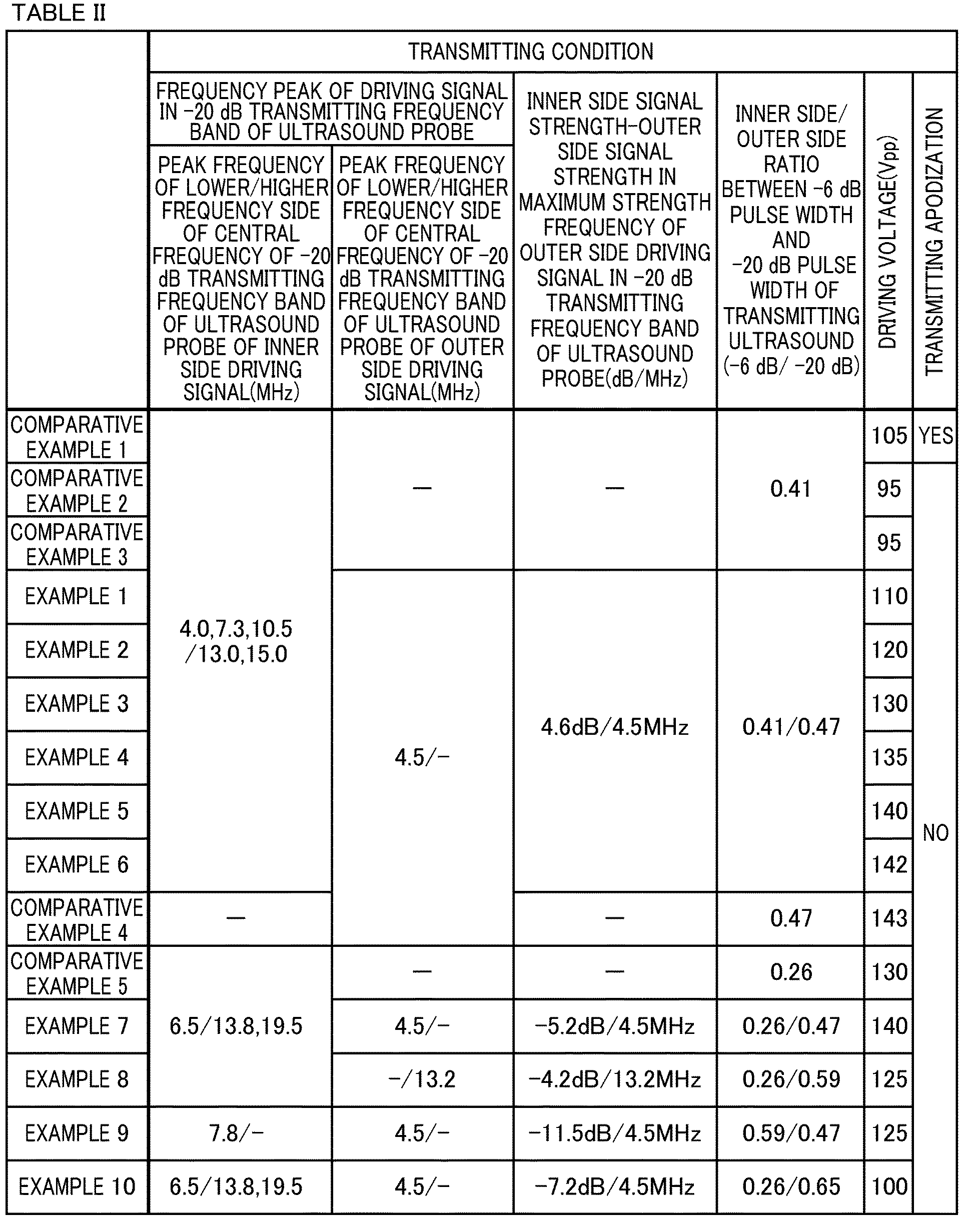

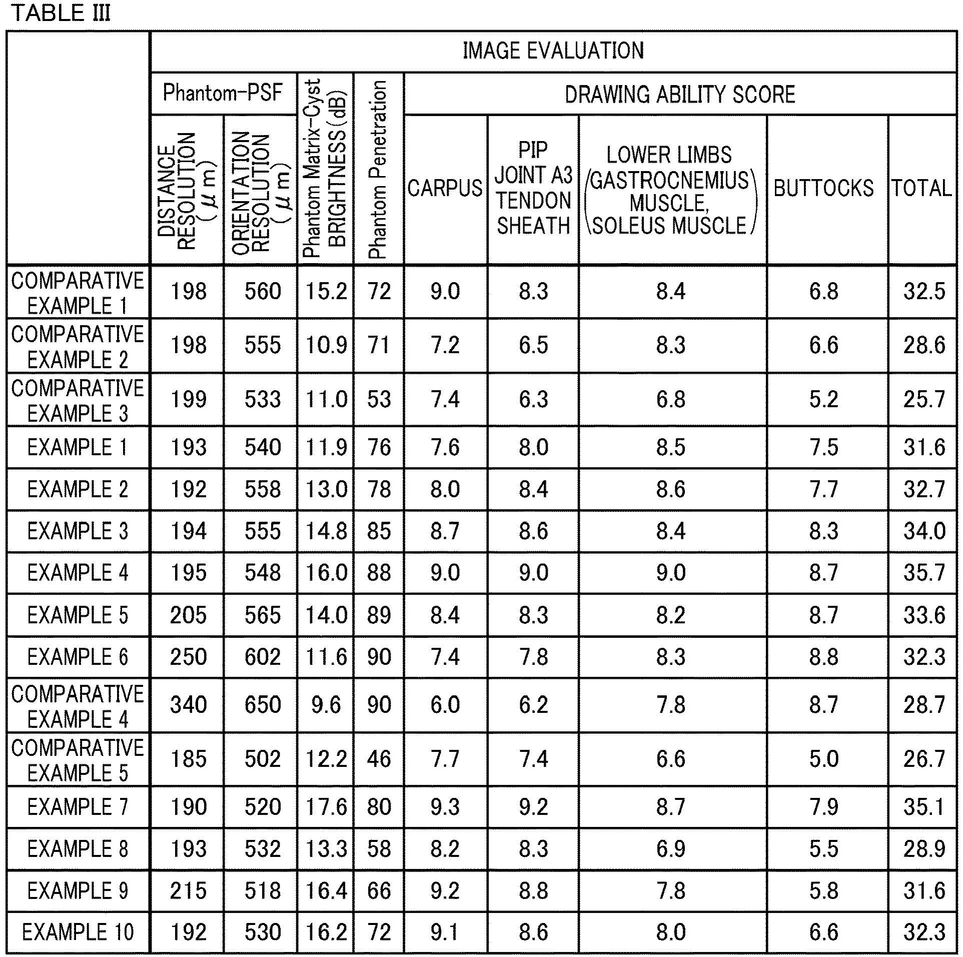

Each item of the transmitting condition in the table I and table II is described. The "inner side channel" in the transmitting condition shows the number of transducers 2a of the inner side element group in the transmitting opening of the ultrasound probe 2 (channel number, upper side), and the percentage [%] of the number of transducers 2a of the inner side element group with respect to the number of transducers 2a of the transmitting opening of the ultrasound probe 2 (lower side). The "outer side channel" in the transmitting condition shows the number of transducers 2a of the outer side element group in the transmitting opening of the ultrasound probe 2 (channel number, upper side), and the percentage [%] of the number of transducers 2a of the outer side element group with respect to the number of transducers 2a of the transmitting opening of the ultrasound probe 2 (lower side).

The "transmitting focal point" in the transmitting condition is the focal point distance [mm] from the ultrasound probe 2 to the focal point of the output transmitting ultrasound. The "driving waveform" in the transmitting condition is the waveform of the driving signal with the waveform different from each other as described above and has the waveforms 1 to 6.

The "inner side driving signal upper limit/ lower limit frequency" in the transmitting condition is, within the -20 dB transmitting frequency band of the ultrasound probe 2, the lower limit/upper limit frequency [MHz] of the -20 dB frequency band (frequency band BO 20 shown in FIG. 9) of the frequency power spectrum of the driving signal input in (the transducer 2a of) the inner side element group of the transmitting opening in the ultrasound probe 2. The "outer side driving signal upper limit/ lower limit frequency" in the transmitting condition is, within the -20 dB transmitting frequency band of the ultrasound probe 2, the lower limit/upper limit frequency [MHz] of the -20 dB frequency band (frequency band BI 20 shown in FIG. 9) of the frequency power spectrum of the driving signal input in the outer side element group of the transmitting opening in the ultrasound probe 2.

The "peak frequency of lower/higher frequency side of central frequency of -20 dB transmitting frequency band of ultrasound probe of inner side driving signal" in the transmitting condition is, within the -20 dB transmitting frequency band of the ultrasound probe 2, the frequency [MHz] of each frequency peak on the lower frequency side and the higher frequency side than the central frequency (central frequency fPC shown in FIG. 8B) of the -20 dB transmitting frequency band of the ultrasound probe 2 regarding the frequency peak of the frequency power spectrum of the driving signal input in the inner side element group of the transmitting opening in the ultrasound probe 2. The "peak frequency of lower/higher frequency side of central frequency of -20 dB transmitting frequency band of ultrasound probe of outer side driving signal" in the transmitting condition is, within the -20 dB transmitting frequency band of the ultrasound probe 2, the frequency [MHz] of each frequency peak (for example, frequency fOP shown in FIG. 8B) on the lower frequency side and the higher frequency side than the central frequency (central frequency fPC shown in FIG. 8B) of the -20 dB transmitting frequency band of the ultrasound probe 2 regarding the frequency peak of the frequency power spectrum of the driving signal input in the outer side element group of the transmitting opening in the ultrasound probe 2.

The "inner side signal strength-outer side signal strength in maximum strength frequency of outer side driving signal in ultrasound probe transmitting sensitivity -20 dB band" in the transmitting condition is, within the - 20 dB transmitting frequency band of the ultrasound probe 2, the signal strength [dB] of the frequency power spectrum of the driving signal input in the inner side element group (signal strength SSI of FIG. 9) - signal strength [dB] of the frequency power spectrum of the driving signal input in the outer side element group (maximum signal strength SSO of FIG. 9) and the frequency [MHz] when the signal strength is maximum when in the frequency that the signal strength of the frequency power spectrum of the driving signal input in the outer side element group of the transmitting opening in the ultrasound probe 2 is maximum (frequency fOP shown in FIG. 9).

Here, FIG. 23 is a schematic diagram showing a power spectrum USSOTx of the transmitting ultrasound transmitted from the inner side element group and a power spectrum USSITx of the transmitting ultrasound transmitted from the outer side element group. The "inner side/outer side ratio between -6 dB pulse width and -20 dB pulse width (-6 dB/-20 dB) of transmitting ultrasound" in the transmitting condition shows the ratio of the -6 dB pulse width with respect to the -20 dB pulse width of the (frequency) power spectrum (power spectrum USSOTx shown in FIG. 23) of the transmitting ultrasound transmitted from the inner side element group of the transmitting opening in the ultrasound probe 2 and the ratio of the -6 dB pulse width with respect to the -20 dB pulse width of the (frequency) power spectrum (power spectrum USSITx shown in FIG. 23) of the transmitting ultrasound transmitted from the outer side element group.

The "driving voltage" in the transmitting condition is the difference between the maximum value and the minimum value [Vpp (Peak to Peak)] of the power supply voltage driving the ultrasound probe 2 and the maximum voltage which is also able to satisfy safety and regulations. According to the present example, bi-polar driving in which positive and negative drives at the same voltage is performed, and 100 Vpp means driving at a maximum of +50V and a minimum of -50V. The "transmitting apodization" in the transmitting condition shows whether transmitting apodization is performed (yes) or not (no).

Each item of the image evaluation result shown in table III is described. The "Phantom-PSF (Point Spread Function)" in the image evaluation result shows a 20 dB resolution (distance resolution [µm], orientation resolution [µm]) as an evaluation result obtained in the image evaluation method in which a stainless wire (SUS) with 50 µm is embedded in a position at a depth of 15mm in an acoustic equivalent material the same as Gammex RMI 404GS- LEO. 5, the ultrasound transmitting, receiving and imaging is performed at a transmitting focal point of 15 mm, the wire drawing brightness of the ultrasound image in the generated image data is converted to an acoustic strength [dB] and the 20 dB resolution is obtained.

The "Phantom-Matrix-Cyst Brightness Difference" in the image evaluation result shows the value (larger is better characteristic) [dB] showing the difference between the acoustic equivalent material average brightness when the average brightness of the 2mm radius region from the center of the 2mmφ non-receiving ultrasound target positioned at the depth of 1cm is measured after adjusting the system gain so that the acoustic equivalent material region average brightness at the depth of 1 cm of the Gammex RMI 404GS-LEO.5 is 80. That is, the "Phantom-Matrix-Cyst brightness difference" maintains the brightness of the signal at a certain value and changes the noise. When the value of the Phantom-Matrix-Cyst brightness difference becomes high, the S/N also becomes high.

The "Phantom-Penetration" in the image evaluation result shows a Penetration depth [mm] as an evaluation result obtained by an image quality evaluation method in which ultrasound transmitting, receiving, and imaging are performed at the transmitting focal point 15 mm, with the acoustic equivalent material of the Gammex RMI 404GS-LEO.5, 2 successive frames of the generated image data are obtained, and the depth that the correlation between the 2 frames of the ultrasound image is lower than 0.5 is obtained as the Penetration depth.

The items of each portion such as "Carpus", "PIP Joint A3 Tendon Sheath", "Lower Limbs (Gastrocnemius Muscle, Soleus Muscle)", "Buttocks", and "Total" in the drawing ability score as the evaluation score of the image evaluation result is described. According to this image quality evaluation method, ultrasound transmitting, receiving and imaging is performed for each portion such as the carpus, PIP joint A3 tendon sheath, lower limbs (gastrocnemius muscle, soleus muscle), and buttocks of the subject, and the drawing of each portion in the ultrasound image of the generated image data is evaluated and scored according to the following evaluation standards by a total of 10 workers related to the orthopedics department including physicians and medical technologists. The scores are averaged (rounded off to one decimal place) for each portion and the drawing ability score as the image quality evaluation result is obtained. The points of the drawing ability score correspond to the following drawing state.
10 = understanding of tissue state is excellent
8 = understanding of tissue state is satisfactory for practical use
6 = understanding of tissue state is not good but enough to understand tissue state
4 = understanding of tissue state is difficult
2 = understanding of tissue state is poor.

Among the above, since the carpus and the PIP joint A3 tendon sheath is positioned in the shallow portion, the resolution of the shallow portion and the S/N can be evaluated. Regarding the carpus, the tubular carpus tube becomes the main target of observation and both distance/orientation resolution and drawing ability of dark portion is necessary to clearly draw this portion. Although orientation resolution is not necessary for drawing the A3 tendon sheath, there is a high demand for the distance resolution and the dark portion drawing ability. Since the depth of the buttocks is deep, it is possible to evaluate the resolution and the S/N for drawing the deep portion and it is necessary that the Phantom-Penetration is high. The desired level of drawing abilities of the dark portion is not high for the lower limbs (gastrocnemius muscle, soleus muscle). However, since the target of observation is from the shallow portion to the deep portion, the evenness and the continuity of the resolution from the shallow portion to the deep portion influences the drawing score.

The "Total" of the drawing ability score is the total value of the drawing ability scores of the "Carpus", "PIP Joint A3 Tendon Sheath", "Lower Limbs (Gastrocnemius Muscle, Soleus Muscle)", and "Buttocks".

Good image evaluation results are obtained for the examples 1 to 10 in which driving signals with different driving waveforms are input in the inner side element group and the outer side element group of the transmitting opening in the ultrasound probe 2 compared to the comparative examples 2 to 5 in which the same driving signal is input in the inner side element group or the outer side element group. Although comparatively good image evaluation results are obtained in the comparative example 1, the transmitting apodization mechanism is necessary and therefore, the costs for the apparatus are high compared to the other examples.

The examples 1 to 7 and 10 in which Triad-THI driving signals are input in the inner side element group of the transmitting opening in the ultrasound probe 2 obtain good image evaluation results compared to the example 9 in which driving signals which are not Triad-THI are input in the inner side element group of the transmitting opening in the ultrasound probe 2. Regarding Triad-THI, for example, according to "Peak Frequency of Lower/Higher Frequency Side of Central Frequency of -20 dB Transmitting Frequency Band of Ultrasound Probe of Inner Side Driving Signal" shown in table II, the frequency power spectrum of the transmitting pulse signal of the driving signal of the inner side element group is a frequency band included in the -20 dB transmitting frequency band of the ultrasound probe 2, there is a strength peak in each of the lower frequency side and the higher frequency side of the central frequency in the transmitting frequency band, and the strength in the frequency region among the plurality of strength peaks is -20 dB or more with the maximum value of the strength of the strength peak as the reference.

According to the examples 1 to 6, the example 2 to 5 with the ratio of the region (channel number) of the inner side element group with respect to the entire transmitting opening in the ultrasound probe 2 being 1/16 to 1/2 has a high value in Phantom-Matrix-Cyst brightness difference (S/N), Phantom Penetration, and image evaluation result. Among the above, especially example 4 obtained the best results. When the ratio of the region (channel number) of the inner side element group with respect to the entire transmitting opening of the ultrasound probe 2 is smaller than 1/16, it becomes difficult to obtain higher harmonic wave generation in the shallow portion by transmitting ultrasound with the inner side element group and the shallow portion S/N decreases (signal component decreases). When the ratio is larger than 1/2, many higher harmonic waves are generated in shallow regions other than near the center of the sound ray, and this is mixed as acoustic noise by scattering, and the dark representation of the shallow portion reduces (increase of noise).

According to the technique of Japanese Patent Application Laid-Open Publication No. 2013-158626, a high resolution can be obtained up to near the transmitting focal point, but the sound pressure near the center of the sound ray drastically decreases in the region deeper than the transmitting focal point. According to the present embodiment, both the inner side element group and the outer side element group transmit ultrasound waves to the same focal point, and both the inner side and the outer side have the similar low frequency component. With this, it is possible to prevent drastic reduction of sound pressure in the region deeper than the transmitting focal point, and as a result, the penetration can be enhanced. In order to prevent the X-type beam profile as shown in FIG. 24C, preferably, in the frequency power spectrum of the transmitting pulse signal of the driving signal of the outer side element group, with respect to the signal strength in the frequency showing the maximum strength in the frequency band included in the -20 dB transmitting frequency band of the ultrasound probe 2, the signal strength with the same frequency in the inner side element group is larger than the signal strength -6 dB of the outer side element group. According to "inner side signal strength-outer side signal strength in maximum strength frequency of outer driving signal in transmitting sensitivity -20 dB band of ultrasound probe", the examples 1 to 8 satisfy the preferable conditions. High penetration is obtained in the examples 1 to 7.

Preferably, as for the ultrasound transmission by the inner side element group according to the present embodiment, broadband transmitting is performed, and more preferably, transmitting as described in FIG. 4A to FIG. 6D is performed. According to the above, it is possible to generate the higher harmonic component to be received only near the center of the sound ray in the shallow portion. When similar broadband transmitting of ultrasound is performed in the outer side element group also, unnecessary higher harmonic waves are generated in the region other than near the center of the sound ray. This decreases the shallow portion image quality by scattering, unnecessary heating increases and the driving voltage cannot be raised. Therefore, preferably, the transmitting ultrasound by the outer side element group easily focuses in the shallow portion, does not include the higher frequency component, and has a relatively narrower band than the transmitting ultrasound by the inner side element group.

Specifically, according to the "inner side driving signal upper limit/lower limit frequency", "outer side driving signal upper limit/ lower limit frequency" as shown in Table II, in the examples 1 to 10, the -20 dB frequency band of the driving signal input in the outer side element group of the transmitting opening in the ultrasound probe 2 in the -20 dB transmitting frequency band of the ultrasound probe 2 is broader than the -20 dB transmitting frequency band of the driving signal input in the inner side element group, that is, the -20 dB frequency band of the driving signal input in the outer side element group is a narrow band. Good image evaluation results are obtained in the examples 1 to 10.

According to the "peak frequency lower/higher frequency side of central frequency of -20dB transmitting frequency band of ultrasound probe of outer side driving signal" as shown in table II, in examples 1 to 7, 9, and 10, in the -20 dB transmitting frequency band of the ultrasound probe 2, the frequency peak of the frequency power spectrum of the driving signal input in the outer side element group of the transmitting opening in the ultrasound probe 2 is in the lower frequency side than the central frequency in the -20 dB transmitting frequency band of the ultrasound probe 2 (not including high frequency component). In example 8, the frequency peak is to the higher frequency side than the central frequency in the -20 dB transmitting frequency of the ultrasound probe 2 (including higher frequency component). The examples 1 to 7, 9, and 10 obtained good image evaluation results compared to example 8.

As shown in FIG. 6A to FIG. 6D, as for the transmitting ultrasound waveform of the inner side element group according to the present embodiment, it is preferable that in the sound wave waveform, the high frequency component is locally concentrated in a relatively short time region and the time of being a large amplitude with respect to the entire pulse width is relatively small, that is, the ratio of -6 dB pulse width/ -20 dB pulse width is small. With this, a multi-step generation of THI (tissue harmonic) as shown in FIG. 6A to FIG. 6D is possible, and the broadband receiving signal can be obtained in both the shallow portion and the deep portion near the center of the sound ray. Turning to the transmitting ultrasound waveform of the outer side region, opposite to the inner side region, it is preferable that the sound wave waveform has an envelope curve shape near the Gaussian, that is, the ratio of -6 dB pulse width/ -20 dB pulse width is relatively large. By transmitting an ultrasound waveform which does not have a locally large amplitude portion, the sound pressure does not rise to the non-linear region in the shallow region far from the sound ray center and the THI is not generated, and the sound pressure rises near the transmitting focal point near the center of the sound ray to the non-linear region and the THI is generated.

By setting the transmitting ultrasound of the inner side element group and the outer side element group as described above, due to the transmitting ultrasound of the inner side element group, as for the shallow portion, the THI is generated only near the center of the sound ray which is the receiving target and the signal strength becomes high, and the THI is not generated in the region far from the sound ray which is not the target of receiving and the acoustic noise due to scattering is not mixed. Therefore, high S/N and good dark portion representation are obtained without using transmitting apodization.

As for the transmitting ultrasound by the outer side element group, the THI unnecessary for imaging is not generated and not attenuated in the shallow region and the THI is generated in the deep region near the focal point. The signal strength in the deep portion is enhanced, and the thermal efficiency is enhanced. With this, the rise of the surface temperature of the ultrasound probe which is the reason for limiting driving voltage is suppressed and penetration is enhanced.

Although the preferable -6 dB pulse width/ -20 dB pulse width cannot be said for sure since this depends on the purpose of the transmitting mode (shallow portion prioritized, deep portion prioritized, balance prioritized, etc.), 0.5 or lower for the inner side element group and 0.4 or higher for the outer side element group is preferable. The preferable relative relation between the inner and outer element group is -6 dB pulse width / -20 dB pulse width ratio of the transmitting ultrasound of the inner side element group < -6 dB pulse width / -20 dB pulse width ratio of the transmitting ultrasound of the outer side element group.

Specifically, according to "inner side/outer side ratio between -6 dB pulse width and -20 dB pulse width of transmitting ultrasound (-6 dB/-20 dB)" shown in table II, the examples 1 to 8 and example 10 satisfies the relation of -6 dB pulse width / -20 dB pulse width ratio of the transmitting ultrasound of the inner side element group < -6 dB pulse width / -20 dB pulse width ratio of the transmitting ultrasound of the outer side element group. Therefore, the values of the Phantom-Matrix-Cyst brightness difference (S/N), Phantom Penetration, and image evaluation results are high. Good S/N, penetration and image evaluation results are obtained.

According to the examples 1 to 10 and comparative examples 1 to 5, as shown in the "driving voltage" of table II, the inner side element group and the outer side element group of the ultrasound probe 2 have a common driving voltage.

As described above, according to the present embodiment, the ultrasound probe 2 includes a transmitting opening including a plurality of transducers 2a. The transmitting opening includes a plurality of transducer groups including a first transducer group and a second transducer group. The ultrasound diagnostic apparatus S includes a transmitter 12 which generates a first driving signal and a second driving signal with a driving waveform different from the first driving signal, provides time delay so that the transmitting ultrasound is focused to the same focal point, outputs the first driving signal to the first transducer group and outputs the second driving signal to the second transducer group. The ultrasound diagnostic apparatus S includes a receiver 13 which receives the receiving signal from the ultrasound probe 2 and the image generator 14 which generates the ultrasound image data from the receiving signal.

More specifically, in the transmitter 12, the first transducer group is the inner side element group positioned on the inner side of the transmitting opening, the second transducer group is the outer side element group positioned on the outer side of the transmitting opening, and the transmitter 12 generates driving signals with different driving waveforms between the inner side element group and the outer side element group.

Therefore, since the transmitting apodization mechanism is not necessary, costs can be suppressed, and the acoustic noise mixing in the shallow portion low receiving ultrasound portion is suppressed without including the transmitting apodization mechanism and good receiving ultrasound image drawing can be obtained. In the region near the focal point and deeper than the transmitting focal point, the higher frequency fundamental wave component of the ultrasound signal transmitted from the inner side element group disappears due to attenuation, and the sound pressure distribution and the beam profile similar to transmitting low frequency fundamental waves from the entire transmitting opening can be obtained. Therefore, since the sound pressure near the center of the beam does not drastically decrease and the higher harmonic wave generated near the focal point propagates mainly near the center, the S/N can be enhanced. It is possible to reduce the unnecessary heat generated by the ultrasound probe 2 due to the acoustic energy which is generated in the region other than the shallow portion receiving region and does not contribute to receiving. This decreases the limitations due to the surface temperature. As a result, the driving voltage can be raised more than conventional examples, and S/N and penetration can be modified and enhanced. The transmitting ultrasound is transmitted to the inner side element group and the outer side element group at the same focal point, and the similar low frequency components are held in both the inner side and the outer side. Therefore, the penetration in the region deeper than the transmitting focal point can be modified and enhanced.

The ultrasound diagnostic apparatus S includes a controller 18 which selects the driving signal from the plurality of driving signals with different driving waveforms for each element group. The transmitter 12 generates the selected driving signal and outputs the driving signal to the element group corresponding to the driving signal. Therefore, the suitable driving signal can be easily selected and generated from the preset plurality of driving signals with different driving waveforms.

Moreover, in the transmitter 12, the -20 dB frequency band of the driving signal in the inner side element group generates the driving signal wider than the -20 dB frequency band of the driving signal in the outer side element group. Therefore, the transmitting ultrasound is transmitted to the inner side element group and the outer side element group at the same focal point, the similar low frequency component can be held in the inner side and the outer side, and the penetration of the region deeper than the transmitting focal point can be modified and enhanced.

The ratio of the -6 dB pulse width with respect to the -20 dB pulse width of the transmitting ultrasound transmitted from the inner side element group is smaller than the ratio of the -6 dB pulse width with respect to the - 20 dB pulse width of the transmitting ultrasound transmitted from the outer side element group. Therefore, in the shallow portion, the THI is generated only near the sound ray center which is the receiving target by the transmitting ultrasound by the inner side element group and the signal strength is enhanced. Therefore, the high S/N and good dark portion representation can be obtained without using the transmitting apodization and the signal strength can be increased in the deep portion. Further, since the heat efficiency is enhanced, the rise in the surface temperature of the ultrasound probe which is the reason for driving voltage being limited can be suppressed, and the penetration can be modified and enhanced.

The transmitter 12 generates a driving signal so that the frequency power spectrum of the transmitting pulse signal of the driving signal of the inner side element group has a frequency band included in the -20 dB transmitting frequency band of the ultrasound probe 2, there is a strength peak in each of the lower frequency side and the higher frequency side than the central frequency of the -20 dB transmitting frequency band, and the strength in the frequency region between the plurality of strength peaks is -20 dB or more with the maximum value of the strength of the strength peak as the reference. Therefore, by realizing the Triad-THI, there is no need to add the complicated circuit to form the waveform of the pulse signal, and the cost can be suppressed while maintaining the high resolution of the transmitting ultrasound. Further, according to the ultrasound image by the fundamental waves, the waveform of the ultrasound with a short pulse and high amplitude can be obtained. Therefore, the low frequency components increase and the penetration can be enhanced while maintaining high resolution.

The transmitter 12 generates a driving signal with the frequency power spectrum of the transmitting pulse signal of the driving signal of the outer side element group having a frequency band included in the -20 dB transmitting frequency band of the ultrasound probe 2, and the maximum strength peak is on the lower frequency side than the central frequency of the -20 dB transmitting frequency band. Therefore, the transmitting ultrasound of the outer side element group is the narrow band not including the high frequency component and easily focuses in the shallow portion. With this, it is possible to prevent the unnecessary higher harmonic wave being generated and scattered in the region other than near the center of the sound ray, to prevent the shallow portion image quality from decreasing and to prevent the unnecessary heat increasing which interferes the raising of the driving voltage. Consequently, the penetration can be enhanced.

The percentage of (the number of channels of the transducers 2a of) the inner side element group with respect to all of the transducers 2a in the transmitting opening is 1/16 to 1/2. Therefore, the higher harmonic wave generating in the shallow portion by ultrasound transmitted by the inner side element group can be obtained and the shallow portion S/N can be enhanced. The higher harmonic wave generating in the shallow portion region other than near the center of the sound ray can be prevented, and the mixing of the acoustic noise can be prevented. With this, the dark portion representation of the shallow portion can be enhanced and the S/N can be enhanced.

The transmitter 12 generates the driving signal so that with respect to the maximum signal strength in the frequency showing the maximum strength in the frequency band included in the -20 dB transmitting frequency band of the ultrasound probe 2 in the frequency power spectrum of the transmitting pulse signal of the driving signal of the outer side element group, the signal strength in the frequency power spectrum of the transmitting pulse signal of the driving signal in the inner side element group with the same frequency as the frequency showing the above maximum strength is larger than the maximum signal strength - 6dB of the driving signal of the outer side element group. Therefore, both the inner side element group and the outer side element group transmit the transmitting ultrasound to the same focal point, and the low frequency component can be held at a similar degree in both the inner side and the outer side. With this, the drastic decrease of sound pressure can be prevented in the region deeper than the transmitting focal point, and the penetration can be modified and enhanced.

The plurality of transducer groups (inner side element group, outer side element group) of the transmitting opening in the ultrasound probe 2 have the common driving voltage. Therefore, it is possible to prevent the complicated configuration of setting different driving voltages for the inner side element group and the outer side element group. Consequently, the configuration can be made simple and the costs can be decreased.

The transmitter 12 and the receiver 13 perform output of the driving signal according to the THI and the generating of the receiving signal. The image generator 14 extracts the higher harmonic wave component from the receiving signal and generates the ultrasound image based on the higher harmonic wave component. Therefore, according to the THI, the higher harmonic wave component is imaged, and the ultrasound image with good contracts can be obtained.

The transmitter 12 and the receiver 13 transmit and receive the ultrasound a plurality of times on the same sound ray by pulse inversion. The image generator 14 extracts the higher harmonic component from the plurality of receiving signals on the same sound ray by the pulse inversion. Therefore, it is possible to obtain the ultrasound image with the unnecessary components attenuated and with high resolution.

The description of the above-described embodiments is one example of the suitable ultrasound diagnostic apparatus and the present invention is not limited to the above.

For example, according to the present embodiment, the transducers 2a of the transmitting opening in the ultrasound probe 2 are divided between the inner side element group and the outer side element group, but the present embodiment is not limited to the above. The transducers 2a of the transmitting opening in the ultrasound probe 2 can be divided into element groups of 3 or more, and the driving signal with the same focal point and different waveform can be selected and input for each element group.

According to the present embodiment, 1 element group (transducer group) includes a plurality of transducers 2a but the present embodiment is not limited to the above. 1 element group may include at least 1 transducer 2a.

The detailed configuration and the detailed operation of each section of the ultrasound diagnostic apparatus S can be suitably modified without leaving the scope of the present invention.

Although embodiments of the present invention have been described and illustrated in detail, the disclosed embodiments are made for purposes of illustration and example only and not limitation. The scope of the present invention should be interpreted by terms of the appended claims.

## Claims

1. An ultrasound diagnostic apparatus (S) which uses an ultrasound probe (2) which transmits transmitting ultrasound to a subject and which receives receiving ultrasound from the subject to generate ultrasound image data from the obtained receiving signal, the apparatus comprising:
the ultrasound probe (2) which includes a transmitting opening including a plurality of transducers, wherein the transmitting opening includes a plurality of transducer groups including at least a first transducer group and a second transducer group;
a transmitter (12) which generates a plurality of driving signals with different driving waveforms including at least a first driving signal and a second driving signal, provides a time delay to the plurality of driving signals so that the transmitting ultrasound focuses to a same focal point, outputs the first driving signal to the first transducer group, outputs the second driving signal to the second transducer group, and outputs the plurality of driving signals to the plurality of transducer groups of the transmitting opening;
a receiver (13) which receives the receiving signal from the ultrasound probe (2); and
an image generator (14) which generates the ultrasound image data from the receiving signal.

2. The ultrasound diagnostic apparatus (S) according to claim 1, further comprising a controller (18) which selects a driving signal from a plurality of driving signals with different driving waveforms for each of the transducer groups,
wherein, the transmitter (12) generates the selected driving signal and outputs the driving signal to the transducer group corresponding to the driving signal.

3. The ultrasound diagnostic apparatus (S) according to claim 1, wherein,
the first transducer group positioned in an inner side of the transmitting opening is to be an inner side transducer group;
the second transducer group positioned in an outer side of the transmitting opening is to be an outer side transducer group; and
the transmitter (12) generates the first driving signal and the second driving signal with a different driving waveform between the first transducer group and the second transducer group.

4. The ultrasound diagnostic apparatus (S) according to claim 3, wherein, the transmitter (12) generates the first driving signal and the second driving signal so that a -20 dB frequency band of the first driving signal is wider than a -20 dB frequency band of the second driving signal.

5. The ultrasound diagnostic apparatus (S) according to claim 3 or 4, wherein, a ratio of a -6 dB pulse width with respect to a -20 dB pulse width of the transmitting ultrasound transmitted from the first transducer group is smaller than a ratio of a -6 dB pulse width with respect to a -20 dB pulse width of the transmitting ultrasound transmitted from the second transducer group.

6. The ultrasound diagnostic apparatus (S) according to any one of claims 3 to 5, wherein, the transmitter (12) generates the first driving signal so that a frequency power spectrum of the transmitting pulse signal of the first driving signal has a frequency band included in a -20dB transmitting frequency band of the ultrasound probe (2), there are strength peaks on each of a lower frequency side than a central frequency of the -20 dB transmitting frequency band and a higher frequency side than the central frequency and the strength in a frequency region between the strength peaks is -20 dB or more with a maximum value of a strength of the strength peak as a reference.

7. The ultrasound diagnostic apparatus (S) according to any one of claims 3 to 6, wherein the transmitter (12) generates the second driving signal so that a frequency power spectrum of the transmitting pulse signal of the second driving signal has a frequency band included in a -20dB transmitting frequency band of the ultrasound probe (2) and a maximum strength peak is on a lower frequency side than a central frequency of the -20 dB transmitting frequency band.

8. The ultrasound diagnostic apparatus (S) according to any one of claims 3 to 7, wherein, a percentage of the first transducer group with respect to all transducers of the transmitting opening is 1/16 to 1/2.

9. The ultrasound diagnostic apparatus (S) according to any one of claims 3 to 8, wherein, the transmitter (12) generates the first driving signal and the second driving signal so that with respect to a maximum signal strength in a frequency showing a maximum strength in a frequency band included in a -20 dB transmitting frequency band of the ultrasound probe (2) in a frequency power spectrum of a transmitting pulse signal of the second driving signal, the signal strength with the frequency in a frequency power spectrum of the transmitting pulse signal of the first driving signal having a same frequency as the frequency showing the maximum strength is larger than the maximum signal strength -6 dB of the second driving signal.

10. The ultrasound diagnostic apparatus (S) according to any one of claims 1 to 9, wherein, the plurality of transducer groups are driven by a common driving voltage.

11. The ultrasound diagnostic apparatus (S) according to any one of claims 1 to 10, wherein,
the transmitter (12) and the receiver (13) output the driving signal and generate the receiving signal according to tissue harmonic imaging; and
the image generator (14) extracts a higher harmonic wave component from the receiving signal and generates an ultrasound image based on the higher harmonic wave component.

12. The ultrasound diagnostic apparatus (S) according to claim 11, wherein,
the transmitter (12) and the receiver (13) transmit and receive ultrasound a plurality of times on a same sound ray by pulse inversion; and
the image generator (14) extracts the higher harmonic wave component from a plurality of receiving signals on the same sound ray by pulse inversion.
